# EUROPEAN PATENT SPECIFICATION

(11) **EP 3 866 821 B1**
(45) Date of publication and mention of the grant of the patent: **28.02.2024**
(21) Application number: 19787257.5
(22) Date of filing: 15.10.2019
(51) Int. Cl.: A61K 35/741, A61P 1/14, A61K 31/19, A61K 45/06, A61K 9/00, A61K 35/747, A61K 35/745, A61K 47/00, A61K 31/11, A61K 35/744, A61K 35/742, A61P 1/12, A61P 35/00, A61K 31/194, A61K 9/02

(54) **CONSORTIA OF LIVING BACTERIA USEFUL FOR TREATMENT OF COLORECTAL CANCER**
KONSORTIEN VON LEBENDEN BAKTERIEN, DIE ZUR BEHANDLUNG VON DARMKREBS GEEIGNET SIND
CONSORTIUM DE BACTÉRIES VIVANTES UTILES POUR LE TRAITEMENT DU CANCER COLORECTAL

(30) Priority: 15.10.2018 EP 18200469
(43) Date of publication of application: 25.08.2021
(73) Proprietor: PharmaBiome AG, 8092 Zürich (CH); Universität Zürich, 8006 Zürich (CH)
(72) Inventor: SCHARL, Michael, Martin, 8904 AESCH (CH); SPALINGER, Marianne, Rebecca, 5603 STAUFEN (CH); DE WOUTERS, Tomas, 8004 ZÜRICH (CH)
(74) Representative: Cabinet Becker et Associés
(86) International application number: PCT/EP2019/078009
(87) International publication number: WO 2020/079024

(56) References cited:
- WO-A1-2012/142605
- WO-A1-2014/110685
- WO-A1-2015/051323
- WO-A1-2016/086161
- US-A1- 2016 000 838
- US-A1- 2016 243 175
- EMMANUELLE ROTH ET AL: "Facultative anaerobic halophilic and alkaliphilic bacteria isolated from a natural smear ecosystem inhibitgrowth in early ripening stages", INTERNATIONAL JOURNAL OF FOOD MICROBIOLOGY, ELSEVIER BV, NL, vol. 147, no. 1, 24 February 2011 (2011-02-24), pages 26-32, XP028197797, ISSN: 0168-1605, DOI: 10.1016/J.IJFOODMICRO.2011.02.032 [retrieved on 2011-03-01]

## Description

### Field

The present invention relates to compositions comprising specific consortia of viable, live bacteria strains and their use to treat colorectal cancer (CRC) and / or intestinal microbiome dysbiosis related to CRC treatment. It was found that the compositions are efficient and safe in these applications.

### Background Art

Colorectal cancer (CRC) is one of the most common forms of cancer. Although current strategies, such as surgery, radiotherapy and chemotherapy have contributed to treatment of CRC, this disease still is a leading cause of cancer-related death. It is known that CRC either develops spontaneously or can be caused by chronic inflammation like IBD. It typically starts with the formation of polyps on the inner lining of the colon or rectum and 95% of CRCs are Adenocarcinomas.

At early stages, local treatment, including surgery and radiation therapy, is the first line of treatment followed by systemic treatment by chemo- or immunotherapy.

Both, CRC and chronic inflammatory diseases of the patients can be caused by a detrimental composition of the gut microbiota, which plays a major role on immune homeostasis. In this context, WO 2016/019506 discloses the use of living bacteria strains from the genera *Eubacterium* in the prevention and treatment for CRC related diseases. Further, WO 2018/112365 discloses the use of living bacteria strains from the family of *Parabacteroides* in the treatment of CRC.

At present, the five-year survival rates for CRC ranges from 90 to 10 % (Stage I to IV, www.cancer.org). It is apparent that there is an unmet clinical need to improve treatment of CRC.

### Summary of the invention

The invention is set out in the appended set of claims.

The present invention relates to a composition comprising viable, live bacteria strains (i), intermediate metabolites (ii), end metabolites (iii), and a dispersing medium (iv), for use as a medicament to treat colorectal cancer (CRC) and / or intestinal microbiome dysbiosis related to CRC treatment, characterized in that said bacteria strains (i) are selected from:
- (A1) strains consuming sugars, fibers, and resistant starch, producing formate and acetate, and being selected from the genera *Ruminococcus, Clostridium, Dorea* and *Eubacterium,* optionally selected from the genera *Ruminococcus, Dorea* and *Eubacterium;*
- (A2) strains consuming sugars, starch and acetate, producing formate and butyrate, and being selected from the genera *Faecalibacterium, Roseburia, Eubacterium* and *Anaerostipes,* optionally selected from the genera *Faecalibacterium, Roseburic* and *Anaerostipes*;
- (A3) strains consuming sugars and oxygen, producing lactate, and being selected from the genera *Lactobacillus, Streptococcus, Escherichia, Lactococcus* and *Enterococcus*;
- (A4) strains consuming sugars, starch, and carbon dioxide, producing lactate, formate and acetate, and being selected from the genera *Bifidobacterium* and *Roseburia,* optionally being of the genus *Bifidobacterium*;
- (A5) strains consuming lactate and proteins, producing propionate and acetate, and being selected from the genera *Clostridium, Propionibacterium, Veillonella, Coprococcus* and *Megasphaera,* optionally selected from the genera *Clostridium, Propionibacterium, Veillonella* and *Megasphaera;*
- (A6) strains consuming lactate and starch, producing acetate, butyrate and hydrogen, and being selected from the genera *Eubacterium, Clostridium* and *Anaerostipes;*
- (A7) strains consuming sugar, starch and formate, producing lactate, formate and acetate, and being selected from the genera *Collinsella* and *Roseburia,* optionally being of the genus *Collinsella*;
- (A8) strains consuming succinate, producing propionate and acetate, and being selected from the genera *Phascolarctobacterium, Flavonifractor* and *Dialister,* optionally selected from the genera *Phascolarctobacterium* and *Dialister*;
- (A9) strains consuming sugars, fibers, formate and hydrogen, producing acetate and optionally butyrate and being selected from the genera *Acetobacterium, Blautia, Clostridium, Moorella, Eubacterium, Methanobrevibacter, Methanomassiliicoccus* and *Sporomusa,* optionally selected from the genera *Acetobacterium, Blautia, Clostridium, Moorella, Methanobrevibacter, Methanomassiliicoccus* and *Sporomusa;* and
- optionally (A10) strains consuming sugars, fibers, and resistant starch, producing succinate, and being selected from the genera *Alistipes, Bacteroides, Blautia, Barnesiella, Clostridium, Ruminococcus* and *Prevotella,* optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*

wherein said bacteria strains are in each case identified through classification of the full 16S gene with assignment for the different taxonomic levels Phylum: 75%, Class: 78.5%, Order: 82%, Family: 86.5%, Genus: 94.5%, sequence similarity;
wherein bacteria strains (i) of all nine groups (A1) to (A9) are present and bacteria strains of group (A10) are optionally present;
wherein said bacteria strains (i) are present in a total concentration of over 10⁹ bacteria per ml composition; and have a viability of over 50%, preferably over 70%, as determined by flow cytometry;
wherein said intermediate metabolites (ii) are selected from: succinate in an amount of less than 5 mM, formate in an amount of less than 5 mM, and lactate in an amount of less than 5 mM; and
wherein said end metabolites (iii) are selected from: acetate in an amount of at least 10 mM, propionate in an amount of at least 2 mM, and butyrate in an amount of at least 2 mM; and wherein said dispersing medium (iv) is preferably selected from: culture media, cryoprotecting media, aqueous gels, and polymeric supports.

In a particular aspect, the composition of the disclosure is for use as a medicament to treat CRC.

In another particular aspect, the composition of the disclosure is for use as a medicament to treat intestinal microbiome dysbiosis related to CRC treatment.

Optionally, said treatment includes the prevention, the treatment, and / or the delay of progression of said disease.

Optionally, the composition is for use as a mono therapy. Alternatively, the composition is for use in combination therapy, in particular in combination with another cancer therapeutic. Said cancer therapeutic is selected from the group of chemotherapeutic agents; cancer immunotherapy agents (particularly checkpoint inhibitors, cancer vaccines, cytokines, cell therapy, CAR-T cells and dendritic cell therapy); angiogenesis inhibitors; and antibiotics.

Optionally, each of said viable, live bacteria strains (i) is present in an amount of 10⁵-10¹⁴ 16S rRNA gene copies per ml, as quantified by qPCR.

Optionally, said composition further comprises one or several bacterial strains selected from
(A11) strains consuming proteins and producing acetate or butyrate, and being selected from the genera *Clostridium, Coprococcus, Eubacterium, Flavonifractor and Flintibacter*;
(A12) strains consuming proteins, fibers, starches or sugars and producing biogenic amines such as y-aminobutyric acid (GABA), cadaverine, dopamine, histamine, putrescine, serotonin, spermidine and/or tryptamine, and being selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers);
(A13) strains consuming primary bile acids and producing secondary metabolites, and being selected from the genera *Anaerostipes, Blautia, Clostridium* and *Faecalibacterium*;
(A14) strains producing vitamins such as cobalamin (B12), folate (B9) or riboflavin (B2), and being selected from the genera *Bacteroides, Bifidobacterium, Blautia, Clostridium, Faecalibacterium, Lactobacillus, Prevotella* and *Ruminococcus*;
(A15) strains consuming mucus and being selected from the genera *Akkermansia, Bacteroides, Bifidobacterium* and *Ruminococcus.*

In one aspect, said viable bacteria strains (i) are such that
- (A1) are selected from *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans and Eubacterium eligens,* optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans and Eubacterium eligens;*
- (A2) are selected from *Faecalibacterium prausnitzii, Anaerostipes hadrus, Roseburia intestinalis Eubacterium ramulus* and *Eubacterium rectale,* optionally selected from *Faecalibacterium prausnitzii, Anaerostipes hadrus,* and *Roseburia intestinalis;*
- (A3) are selected from *Lactobacillus rhamnosus, Streptococcus salivarius, Escherichia coli, Lactococcus lactis, Enterococcus faecalis* and *Enterococcus caccae,* optionally selected from *Lactobacillus rhamnosus, Streptococcus salivarius, Escherichia coli, Lactococcus lactis* and *Enterococcus caccae*;
- (A4) are selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum,* and *Roseburia hominis,* optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum* and *Bifidobacterium pseudocatenulatum*;
- (A5) are selected from *Clostridium aminovalericum, Clostridium celatum, Clostridium lactatifermentans, Clostridium neopropionicum, Clostridium propionicum, Megasphaera elsdenii, Veillonella montpellierensis, Coprococcus catus* and *Veillonella ratti,* optionally selected from *Clostridium aminovalericum, Clostridium celatum, Clostridium lactatifermentans, Clostridium neopropionicum, Clostridium propionicum, Megasphaera elsdenii, Veillonella montpellierensis* and *Veillonella ratti;*
- (A6) are selected from *Anaerostipes caccae, Clostridium indolis, Eubacterium hallii, Eubacterium limosum,* and *Eubacterium ramulus;*
- (A7) are selected from *Collinsella aerofaciens, Collinsella intestinalis, Collinsella stercoris, and Roseburia hominis,* optionally selected from *Collinsella aerofaciens, Collinsella intestinalis* and *Collinsella stercoris*;
- (A8) are selected from *Phascolarctobacterium faecium, Dialister succinatiphilus, Flavonifractor plautii* and *Dialister propionifaciens,* optionally selected from *Phascolarctobacterium faecium, Dialister succinatiphilus* and *Dialister propionifaciens*;
- (A9) are selected from *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii* and *Candidatus Methanomassiliicoccus intestinalis,* optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii* and *Candidatus Methanomassiliicoccus intestinalis; and* / *or*
- (A10) are selected from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii,* optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii,* preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii,* and *Alistipes shahii.*

In a particular aspect, said viable bacteria strains (i) are *Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6), *Collinsella aerofaciens* (A7), *Phascolarctobacterium faecium* (A8), and *Blautia hydrogenotrophica* (A9) and optionally *Bacteroides xylanisolvens* (A10).

In another particular aspect, said viable bacteria strains (i) are *Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6 and A9), *Collinsella aerofaciens* (A7) and *Phascolarctobacterium faecium* (A8) and optionally *Bacteroides xylanisolvens* (A10).

Optionally, component (iv) is selected from: cryoprotecting media comprising glycerol; and/or culture media comprising peptone, yeast extract, monosaccharides, disaccharides, arabinogalactan, fructo-oligosaccharides, fibres, glycerol, soluble starch, resistant starch, xylan, minerals, co-factors, vitamins and reducing agents.

Optionally, the composition is free of, or essentially free of, other viable, live bacteria.

Optionally, the composition is free of, or essentially free of, succinate, formate and lactate.

Optionally, the composition is adapted to rectal administration.

### Optionally, the composition is adapted to oral administration. Figures

The present invention will be better understood by reference to the **figures.**

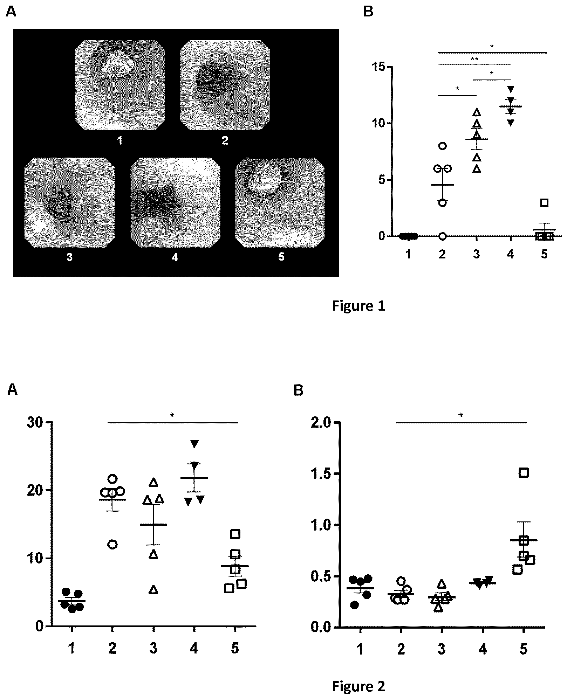

Fig. 1: Assessment of tumor counts in C57/B6 mice that were subjected to three cycles of dextran sulfate sodium (DSS) treatment (7 days with 2% DSS in the drinking water, followed by 10 days of recovery, each). At day 1 and day 9 of each DSS cycle, mice received 10 mg/kg Azoxymethane (AOM). Treatments, consisting of viable live bacteria were gavaged once daily at days 7 - 9 of each AOM-DSS cycle.

Treatment groups represent the negative control treated with DSS but no AOM (1), the positive control gavaged with PBS (2), the group treated with *Akkermansia muciniphila* (3); the group treated with *Peptostreptococcus stomatis* (4) and the group treated with PB002 (5); cf. example 3.

Three weeks after the last AOM-DSS cycle, tumor load was determined by mouse endoscopy (A), and tumors counted in resected and opened colons (B) whereby the y-axis corresponds to the number of tumors per mouse. Statistical analysis was performed using unpaired Mann-Whitney U test: *: p < 0.05, **: p <0.01, ***: p < 0.005.

It was shown that treatment groups (3) and (4) promote tumor growth, whereas treatment group (5) showed significantly lower tumor counts compared to treatment group (2).

Fig. 2: Quantification of intestinal immune response in C57/B6 mice that were treated as described in Fig. 1, cf. example 4. At the end of the experiment, lamina propria immune cells were harvested and analyzed using flow cytometry. The graphs show (A) relative proportion of F4/80+ macrophages (frequency in CD45+ and CD3- cells) and (B) relative proportion of NK1.1 positive NK cells (frequency of CD45+ cells). Statistical analysis was performed using unpaired Mann-Whitney U test: *: p < 0.05, **: p <0.01, ***: p < 0.005.

The experiments showed a specific induction of NK and Th1 type cells in the lamina propria of the PB002 treated mice. NK and Th1 type immune cells are commonly known to promote the clearance of the tumor cell by the immune system thereby preventing expansion of cancer.

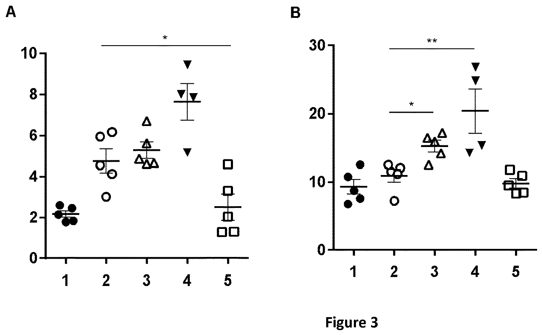

Fig. 3: Quantification of intestinal immune response in C57/B6 mice that were treated as described in figure 1, cf. example 4. Three weeks after the last AOM-DSS cycle, colonic lamina propria immune cells were isolated and frequency of (A) PD-L1+ non-T cells (% of CD45+CD3- cells), as well as (B) PD1+ CD8+ T-cells (% of CD45+CD3CD8+ cells) analyzed using flow cytometry. Statistical analysis was performed using unpaired Mann-Whitney U test: *: p < 0.05, **: p <0.01, ***: p < 0.005. PB002 suppressed the PD1 and PD1-L activation reinforcing the immune clearance of potential tumors. The PD1 suppressor system is commonly known to favor tumor growth. It is further known, that suppression of PD1 can reinforce immune clearance as repeatedly shown using PD1 inhibitors as cancer therapeutics.

### Description of the Invention

Thus, it is an object of the present invention to mitigate at least some of these drawbacks of the state of the art. In particular, it is an aim of the present invention to provide compositions useful treat CRC and / or intestinal microbiome dysbiosis related to CRC treatment.

These objectives are achieved by compositions and methods as outlined in the specification and defined in the independent claims. Preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

### Definitions

Unless otherwise stated, the following **definitions** shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, nonlimiting sense.

As used herein, the term "consist essentially of" or "essentially consist" refers to those elements required for a given embodiment. This term indicate the inclusion of any recited characteristics and permits the optional presence of elements that do not materially affect nor change the characteristics or functions of said embodiment. Preferably, in the context of a composition comprising bacteria, it refers to a composition that comprises the recited bacteria, and optionally includes other components such as prebiotics, at the exclusion of non-recited bacteria.

The term "Microbiota" is known and particularly denotes the totality of microbial life forms within a given habitat or host.

The term "Dysbiosis" is known and denotes the alteration of the microbiota in comparison to the healthy state. The microbiota's state may be characterized by determining key markers, intermediate metabolites and end metabolites. The healthy microbiota is characterized by the absence of intermediate metabolites. Accordingly, a stable state characterized by accumulation of intermediate metabolites is referred to as dysbiosis.

The term "Intermediate Metabolite" denotes the metabolites produced by members of the microbiota that are used as energy source by other members of the microbiota. Such intermediate metabolites are typically not enriched in the feces of a healthy individual. Such intermediate metabolites in particular may include degradation products from fibers, proteins or other organic compounds, but also formate, lactate and succinate. More generally, the term "intermediate metabolites" may refer to an undesirable metabolite, the presence or amount of which being limited as much as possible in the final product and/or patient.

The term "End Metabolites" denotes the metabolites produced by the intestinal microbiota that are not or only partially utilized by other members of the microbiota. End metabolites are partially absorbed by the host and partially secreted in the feces. End metabolites in particular include the short chain fatty acids acetate, propionate and butyrate comprising two, three and four carbon atoms, respectively.

The term "Effluent" is known and particularly denotes the outflow of a continuous fermentation process containing consumed growth medium, bacteria and bacterial metabolites.

The terms "Bacteria" and "Bacteria Strain" are known and particularly denote the totality of the domain *Bacteria.* Due to its function, also the genera *Methanobrevibacter* and *Candidatus Methanomassiliicoccus* of the Domain *Archaea* shall be included in the term "Bacteria".

It should be noticed that bacterium *Clostridium lactatifermentans* has been recently renamed *Anaerotignum lactatifermentans.* Then, as used herein the terms *"Clostridium lactatifermentans"* and *"Anaerotignum lactatifermentans"* have the same meaning and can be used interchangeably.

The terms "viable bacteria" and "live bacteria" are known in the field; in particular they denote bacteria, whereby viable bacteria strains have the capacity to grow under suitable conditions and live bacteria indicate viability as measured using biochemical assays.

The term "consortium", "microbial consortium" or "bacterial consortium" refers herein to at least three microbial organisms, preferably officiating in the same metabolic or trophic network. As such, microbial members of the consortium collaborate, preferably for their subsistence into the consortium.

The term "functional group" as used herein, refers to functions or capacities fulfilled by bacteria. Such functions are for example capacity to degrade or convert a particular substrate, for example such as starch, and to produce a particular product or metabolite, for example such as butyrate. Generally, one bacterium is able to degrade or convert a substrate (e.g. starch) and to produce a product (e.g. butyrate). Then, a functional group comprises bacteria that are able to degrade or convert the same substrate(s) (e.g. starch) and to produce the same metabolite(s) (e.g. butyrate).

The term "cancer" broadly refers to an uncontrolled, abnormal growth of a host's own cells leading to invasion of surrounding tissue and potentially tissues distal to the initial site of abnormal cell growth in the host.

Accordingly, the expressions "cancer treatment related diseases" and "related to cancer treatments" are generally understood and refer to disorders iatrogenic to cancer treatment. The expression specifically describes the occurrence of a disease as side effects of cancer treatments

The terms "dispersing medium", "cultivation medium" and "culture medium" are used interchangeably herein and refer to a liquid or solid medium in which the bacterial strains are inoculated and/or cultivated.

In general terms, the invention relates to compositions comprising viable, live bacteria strains (i), intermediate metabolites (ii), end metabolites (iii), and a dispersing medium (iv), each as defined herein, which are useful to treat CRC and / or intestinal microbiome dysbiosis related to CRC treatment.

Accordingly, these compositions are useful in pharmaceutical applications as described herein. Due to the function-based selection of the bacteria strains in the compositions, a very low maximum amount of undesired intermediate metabolites (ii) and a very high amount of desired end metabolites (iii), representing physiologically relevant ratios, is ensured.

These compositions comprise specific viable, live bacteria strains (i), intermediate metabolites to a maximum concentration, (ii) end metabolites (iii) to a specific minimum concentration and optionally a dispersing medium (iv) as defined below.

These compositions are obtainable as described below providing a stable, highly concentrated consortium of viable bacteria.

Hereinafter, the pharmaceutical applications, the compositions and their manufacturing shall be explained in more detail.

### 1. Pharmaceutical Applications

The compositions described herein are useful as pharmaceuticals in the applications described herein. Particularly, the compositions described herein are pharmaceutical compositions. As used herein, a "pharmaceutical composition" may refer to a preparation of one or more of the active agents, such as comprising a consortium according to the invention, with optional other chemical components such as physiologically suitable carriers and excipients.

Compositions described herein can be in a form suitable for any conventional route of administration or use. In one embodiment, a "composition" typically intends a combination of the active agent, e.g., compound or composition, and a naturally-occurring or non-naturally-occurring carrier, inert (for example, a detectable agent or label) or active, such as an adjuvant, diluent, binder, stabilizer, buffers, salts, lipophilic solvents, preservative, adjuvant or the like and include pharmaceutically acceptable carriers. An "acceptable vehicle" or "acceptable carrier" as referred to herein, is any known compound or combination of compounds that are known to those skilled in the art to be useful in formulating pharmaceutical compositions.

The compositions described herein may be applied to any subject in need thereof, including mammals, particularly human beings.

In one embodiment, the compositions are used to treat CRC.

In one further embodiment, the compositions are used to treat intestinal microbiome dysbiosis related to CRC treatment. As used herein, the expression "intestinal microbiome dysbiosis related to CRC treatment" includes any type of microbial un-balance caused directly or indirectly by the treatment of CRC. Without being bound to theory, it is believed that microbiome dysbiosis is a consequence of CRC treatments. Typically, the symptoms are related to a loss of the barrier function of the intestinal epithelium and a consequent infiltration of bacteria or bacterial components, leading to an immune reaction and to acute or chronic inflammation, such as, but not exclusively, colitis.

In one embodiment, the invention provides for a composition as described herein for use as a medicament to treat CRC and/or intestinal microbiome dysbiosis related to CRC treatments.

Accordingly, the specification also discloses the use of a composition as described herein for the manufacturing of a medicament to treat CRC and/or intestinal microbiome dysbiosis related to CRC treatments.

Still further, the specification discloses a method for treating CRC and/or intestinal microbiome dysbiosis related to CRC treatments in a subject in need thereof, said method comprising the step of administering a sufficient or therapeutically effective amount of a composition as described herein to a subject in need thereof.

A "therapeutically effective amount" or "sufficient amount" is an amount which, when administered to a subject, is the amount of active agent that is needed to treat the targeted disease or disorder, or to produce the desired effect. The therapeutic agent(s), the disease and its severity, the physiological data and characteristics of the patient or subject to be treated (e.g. age, size, and weight), and the routes of administration have to be taken into account to determine the appropriate dosage, so as a therapeutically effective amount will be administered to the patient or subject.

As used herein, the term "treatment" also includes the prevention of diseases described herein and the delay of progression of diseases described herein. Particularly, the term "treatment" refers to any act intended to ameliorate the health status of patients or subjects such as therapy, prevention, prophylaxis and retardation of a disease. It designates both a curative treatment and/or a prophylactic treatment of a disease. A curative treatment is defined as a treatment resulting in cure or a treatment alleviating, improving and/or eliminating, reducing and/or stabilizing the symptoms of a disease or the suffering that it causes directly or indirectly. A prophylactic treatment comprises both a treatment resulting in the prevention of a disease and a treatment reducing and/or delaying the incidence of a disease or the risk of its occurrence. In certain embodiments, such term refers to the improvement or eradication of a disease, a disorder or symptoms associated with it.

As used herein, the term "subject", "host", "individual," or "patient" refers to human and veterinary subjects particularly to an animal, preferably to a mammal, even more preferably to a human, including adult and child. However, the term "subject" also encompasses non-human animals, in particular mammals such as dogs, cats, horses, cows, pigs, sheep and non-human primates, among others.

The subject to treat according to the invention is an animal, preferably a mammal, even more preferably a human. However, the term "subject" can also refer to non-human animals, in particular mammals such as dogs, cats, horses, cows, pigs, sheep, donkeys, rabbits, ferrets, gerbils, hamsters, chinchillas, rats, mice, guinea pigs and non-human primates, among others, or non-mammals such as poultry, that are in need of treatment. Preferably, the subject is a human.

Optionally, the treatment is administered to the subject regularly, preferably between every day and every month, more preferably between every day and every two weeks, more preferably between every day and every week, even more preferably the treatment is administered every day. In a particular embodiment, the treatment is administered several times a day, preferably 2 or 3 times a day, even more preferably 3 times a day.

### 2. Mono Therapy and Combination Therapy

As used herein, the term "treatment" includes both, stand-alone-treatment ("mono therapy") and treatment in combination with other cancer therapeutics ("combination therapy"). The term "in combination" as used herein refers to the use of more than one therapy and does not restrict the order in which therapies are administered to a subject.

Accordingly, the invention provides for a composition as described herein for use as a mono therapy. Such mono therapies are also referred to as "stand-alone treatment".

Further, the invention provides for a composition as described herein for use in combination with one or more further cancer therapeutics, and/or with other components or cell populations that are preferably immunostimulatory. Combination therapy shall include consecutive and simultaneous administration of the compositions described herein with one or more cancer therapeutics. Suitable cancer therapeutics are known per se and are preferably selected from the group of
1. chemotherapeutic agents;
2. cancer immunotherapy agents (particularly checkpoint inhibitors, cancer vaccines, cytokines, cell therapy, CAR-T cells and dendritic cell therapy)
3. angiogenesis inhibitors; and
4. antibiotic agents.

Such other cancer therapeutics are outlined in further detail below (section No. 5).

### 3. Compositions

Some compositions described herein are known and disclosed in WO2018189284.

### Component (i), viable, live bacteria strains:

The term viable, live bacteria strains is known in the field and particularly relates to bacteria strains (i) having a viability of over 50% (e.g. in pharmaceutical products), typically over 60% such as over 90% (e.g. products obtained according to the inventive process) as determined by flow cytometry. Viability over 90% is typically observed from the compositions as initially obtained by continuous cultivation, viability over 60% is typically observed after stabilization or preservation.

Bacteria strains useful in the context of this invention are described below as (A1) to (A**), preferably (A1) to (A9), optionally in combination with (A10), (A11), (A12), (A13), (A14) and/or (A15). The concentration of these bacteria strains (i) in the inventive compositions may vary over a broad range, typically the total concentration of all bacteria strains (A1) to (A**), preferably (A1) to (A9), optionally in combination with (A10), (A11), (A12), (A13), (A14) and/or (A15), is above 10⁹ bacteria per ml composition, as determined by quantitative polymerase chain reaction (qPCR).

Fiber degradation by bacterial fermentation in the intestine is the central function of the intestinal microbiome (cf. Lacroix and Chassard (2013)). It is generally known that the metabolite concentrations obtained from fiber degradation are independent of the composition of the recipient's microbiome. It was surprisingly found that the effect of the inventive compositions is independent of the concentration of end metabolites and undesired intermediate metabolites in the gut of the recipient upon administration. Though present in most individuals, the single metabolic interactions are known to vary in their relative abundance between individuals. *Bacteroidetes* dominated microbiomes might enrich succinate while *Ruminococcus* enriched microbiomes might enrich formate upon degradation of fibers. The inventive compositions take into account the possible variations in different microbiome compositions in the patient population and re-balance dysbiosis independent of the nature thereof.

The inventive compositions comprise bacteria strains of groups (A1) to (A9) as defined herein, and optionally of groups (A10), (A11), (A12), (A13), (A14) and/or (A15). The presence of all 9 groups is important to ensure the beneficial pharmaceutical effects described below. Accordingly, groups (A1) to (A9) are present in the inventive compositions, further groups may be present as well, but are not required. In one embodiment, bacteria strains of groups (A1) to (A9) are present; further groups of bacteria strains being absent. In one further embodiment, bacteria strains of groups (A1) to (A9) are present; one or more further groups of bacteria strains (A10) to (A**), especially (A10), (A11), (A12), (A13), (A14) and/or (A15), being additionally present. Such further bacteria strains may be beneficial, e.g. to improve stability of the consortium.

In a particular aspect, each of the bacteria belongs to at least one functional group but to no more than 2, 3, 4 or 5 functional groups. This means that a particular bacterial strain can belong to more than one functional group, for example to two or three functional groups. Preferably, a particular bacterial strain cannot belong to all of the functional groups (A1-A**), especially (A1)-(A9). In another particular embodiment, each of the functional groups comprises only one bacterial strain. Alternatively, the functional groups comprise more than one bacterial strain. As discussed below, all bacteria groups are defined by their functions. Such functions may be accomplished by one or more than one bacteria strain. Accordingly, each group comprises one or more, preferably one, bacteria strain.

In view of the intended use, bacteria strains (A1) to (A**), in particular (A1) to (A15), preferably belong to the group of intestinal bacteria strains.

As discussed below, the concentration of bacteria strains according to groups (A1) to (A**), in particular (A1) to (A15), may vary over a broad range. Typically, each group is present in a concentration below 10¹⁴16S rRNA gene copies per ml composition. Typically, each group is present in a concentration above 10⁵ 16S rRNA gene copies per ml composition, preferably above 10⁶ 16S rRNA gene copies per ml composition, particularly preferably above 10⁸ 16S rRNA gene copies per ml composition. Preferably, the concentration of bacteria strains is quantified by qPCR.

The bacteria strains are obtainable by co-cultivation, see #3. This co-cultivation ensures a balanced amount of each of the strains and the establishment of a metabolic interaction, thereby providing a synergistic interaction resulting in a higher amount of bacteria strains (A1) to (A**), particularly (A1) to (A9), optionally in combination with (A10), (A11), (A12), (A13), (A14) and/or (A15), and an increased robustness of the single strains and the mixes thereof.

**Group (A1)** comprises bacteria strains consuming sugars, fibers, and resistant starch, producing formate and acetate. Such bacteria strains are known and include bacteria of the genera *Ruminococcus, Clostridium, Dorea* and *Eubacterium,* such as the species *Ruminococcus bromii* (ATCC 27255, ATCC 51896), *Ruminococcus lactaris* (ATCC 29176), *Ruminococcus chomponellensis* (DSM 18848, JCM 17042), *Ruminococcus callidus* (ATCC 27760), *Ruminococcus gnavus* (ATCC 29149, ATCC 35913, JCM 6515), *Ruminococcus obeum* (ATCC 29174, DSM 25238, JCM 31340), *Dorea longicatena* (DSM 13814, JCM 11232), *Dorea formicigenerans* (ATCC 27755, DSM 3992, JCM 31256), *Clostridium scindens (DSM 5676, ATCC 35704) and Eubacterium eligens* (ATCC 27750, DSM 3376).

Optionally, **Group (A1)** comprises bacteria strains consuming sugars, fibers, and resistant starch, producing formate and acetate. Such bacteria strains are known and include bacteria of the genera *Ruminococcus, Dorea* and *Eubacterium* such as the species *Ruminococcus bromii* (ATCC 27255, ATCC 51896), *Ruminococcus lactaris* (ATCC 29176), *Ruminococcus chomponellensis* (DSM 18848, JCM 17042), *Ruminococcus callidus* (ATCC 27760), *Ruminococcus gnavus* (ATCC 29149, ATCC 35913, JCM 6515), *Ruminococcus obeum* (ATCC 29174, DSM 25238, JCM 31340), *Dorea longicatena* (DSM 13814, JCM 11232), *Dorea formicigenerans* (ATCC 27755, DSM 3992, JCM 31256) and *Eubacterium eligens* (ATCC 27750, DSM 3376).

**Group (A2)** comprises bacteria strains consuming sugars, starch and acetate, and producing formate and butyrate. Such bacteria strains are known and include bacteria of the genera *Faecalibacterium, Roseburia, Eubacterium* and *Anaerostipes* such as the species *Faecalibacterium prausnitzii* (ATCC 27768, ATCC 27766, DSM 17677, JCM 31915), *Anaerostipes hadrus* (ATCC 29173, DSM 3319), *Roseburia intestinalis* (DSM 14610, CIP 107878, JCM 31262), *Eubacterium ramulus* (ATCC 29099, DSM 15684, JCM 31355) and *Eubacterium rectale* (DSM 17629).

Optionally, group (A2) comprises bacteria strains consuming sugars, starch and acetate, and producing formate and butyrate. Such bacteria strains are known and include bacteria of the genera *Faecalibacterium, Roseburia* and *Anaerostipes* such as the species *Faecalibacterium prousnitzii* (ATCC 27768, ATCC 27766, DSM 17677, JCM 31915), *Anaerostipes hadrus* (ATCC 29173, DSM 3319) and *Roseburia intestinalis* (DSM 14610, CIP 107878, JCM 31262).

**Group (A3)** comprises bacteria strains consuming sugars and oxygen, producing lactate. Such bacteria strains are known and include bacteria of the genera *Lactobacillus, Streptococcus, Escherichia, Lactococcus, Enterococcus* such as the species *Lactobacillus rhamnosus* (ATCC 7469, DSM 20021, JCM 1136), *Streptococcus salivarius* (ATCC 7073, DSM 20560, JCM 5707), *Escherichia coli* (ATCC 11775, DSM 30083, JCM 1649), *Lactococcus lactis* (ATCC 19435, DSM 20481), *Enterococcus caccae* (ATCC BAA-1240, DSM 19114), and *Enterococcus faecalis* (ATCC 29212, DSM 2570). Optionally, the bacteria strains are selected from the species *Lactobacillus rhamnosus, Streptococcus salivarius, Escherichia coli, Lactococcus lactis* and *Enterococcus caccae.*

**Group (A4)** comprises bacteria strains consuming sugars, starch, and carbon dioxide, producing lactate, formate and acetate. Such bacteria strains are known and include bacteria of the genus *Bifidobacterium* and *Roseburia,* such as the species *Bifidobacterium adolescentis* (ATCC 15703, DSM 20083, JCM 1251), *Bifidobacterium angulatum* (ATCC 27535, DSM 20098), *Bifidobacterium bifidum* (ATCC 29521, DSM 20456, JCM 1255), *Bifidobacterium breve* (ATCC 1192, DSM 20213), *Bifidobacterium catenulatum* (ATCC 27539, DSM 16992, JCM 1194), *Bifidobacterium dentium* (ATCC 27534, DSM 20436, JCM 1195), *Bifidobacterium gallicum* (ATCC 49850, DSM 20093, JCM 8224), *Bifidobacterium longum* (ATCC 15707, DSM 20219, JCM 1217), *Bifidobacterium pseudocatenulatum* (ATCC 27919, DSM 20438, JCM 1200) and *Roseburia hominis (DSM 16839).*

Optionally, group (A4) comprises bacteria strains consuming sugars, starch, and carbon dioxide, producing lactate, formate and acetate. Such bacteria strains are known and include bacteria of the genus *Bifidobacterium,* such as the species *Bifidobacterium adolescentis* (ATCC 15703, DSM 20083, JCM 1251), *Bifidobacterium angulatum* (ATCC 27535, DSM 20098), *Bifidobacterium bifidum* (ATCC 29521, DSM 20456, JCM 1255), *Bifidobacterium breve* (ATCC 1192, DSM 20213), *Bifidobacterium catenulatum* (ATCC 27539, DSM 16992, JCM 1194), *Bifidobacterium dentium* (ATCC 27534, DSM 20436, JCM 1195), *Bifidobacterium gallicum* (ATCC 49850, DSM 20093, JCM 8224), *Bifidobacterium longum* (ATCC 15707, DSM 20219, JCM 1217), and *Bifidobacterium pseudocatenulatum* (ATCC 27919, DSM 20438, JCM 1200).

**Group (A5)** comprises bacteria strains consuming lactate and proteins, producing propionate and acetate. Such bacteria strains are known and include bacteria of the genera *Clostridium, Propionibacterium, Veillonella, Megasphaera* and *Coprococcus* such as the species *Clostridium aminovalericum* (ATCC 13725, DSM 1283, JCM 1421), *Clostridium celatum* (ATCC 27791, DSM 1785, JCM 1394), *Clostridium lactatifermentans* (DSM 14214), *Clostridium neopropionicum* (DSM 3847), *Clostridium propionicum* (ATCC 25522, DSM 1682, JCM 1430), *Megasphaera elsdenii* (ATCC 25940, DSM 20460, JCM 1772), *Veillonella montpellierensis* (DSM 17217), *Veillonella ratti* (ATCC 17746, DSM 20736, JCM 6512) and *Coprococcus catus (ATCC 27761).*

Optionally, group (A5) comprises bacteria strains consuming lactate and proteins, producing propionate and acetate. Such bacteria strains are known and include bacteria of the genera *Clostridium, Propionibacterium, Veillonella, Megasphaera* such as the species *Clostridium aminovalericum* (ATCC 13725, DSM 1283, JCM 1421), *Clostridium celatum* (ATCC 27791, DSM 1785, JCM 1394), *Clostridium lactatifermentans* (DSM 14214), *Clostridium neopropionicum* (DSM 3847), *Clostridium propionicum* (ATCC 25522, DSM 1682, JCM 1430), *Megasphaera elsdenii* (ATCC 25940, DSM 20460, JCM 1772), *Veillonella montpellierensis* (DSM 17217), and *Veillonella ratti* (ATCC 17746, DSM 20736, JCM 6512).

**Group (A6)** comprises bacteria strains consuming lactate and starch, producing acetate, butyrate and hydrogen. Such bacteria strains are known and include bacteria of the genera *Anaerostipes, Clostridium,* and *Eubacterium* such as the species *Anaerostipes caccae* (DSM 14662, JCM 13470), *Clostridium indolis* (ATCC 25771, DSM 755, JCM 1380), *Eubacterium hallii* (ATCC 27751, DSM 3353, JCM 31263), *Eubacterium limosum* (ATCC 8486, DSM 20543, JCM 6421), *Eubacterium ramulus* (ATCC 29099, DSM 15684, JCM 31355).

**Group (A7)** comprises bacteria strains consuming sugar, starch and formate, producing lactate, formate and acetate. Such bacteria strains are known and include bacteria of the genus *Collinsella* and *Roseburia,* such as the species *Collinsella aerofaciens* (ATCC 25986, DSM 3979, JCM 10188), *Collinsella intestinalis* (DSM 13280, JCM 10643), *Collinsella stercoris* (DSM 13279, JCM 10641) and *Roseburia hominis* (DSM 16839).

Optionally, group (A7) comprises bacteria strains consuming sugar, starch and formate, producing lactate, formate and acetate. Such bacteria strains are known and include bacteria of the genus *Collinsella,* such as the species *Collinsella aerofaciens* (ATCC 25986, DSM 3979, JCM 10188), *Collinsella intestinalis* (DSM 13280, JCM 10643) and *Collinsella stercoris* (DSM 13279, JCM 10641).

**Group (A8)** comprises bacteria strains consuming succinate, producing propionate and acetate. Such bacteria strains are known and include bacteria of the genera *Phascolarctobacterium, Dialister* and *Flavonifractor such* as the species *Phascolarctobacterium faecium* (DSM 14760), *Dialister succinatiphilus* (DSM 21274, JCM 15077), *Dialister propionifaciens* (JCM 17568) and *Flavonifractor plautii* (ATCC 29863, DSM 4000).

Optionally, group (A8) comprises bacteria strains consuming succinate, producing propionate and acetate. Such bacteria strains are known and include bacteria of the genera *Phascolarctobacterium, Dialister* such as the species *Phascolarctobacterium faecium* (DSM 14760), *Dialister succinatiphilus* (DSM 21274, JCM 15077) and *Dialister propionifaciens* (JCM 17568).

**Group (A9)** comprises bacteria strains consuming sugars, fibers, formate and hydrogen, producing acetate and optionally butyrate. Such bacteria strains are known and include bacteria of the genus *Acetobacterium, Blautia, Clostridium, Moorella, Sporomusa* and *Eubacterium* and archaea of the genera *Methanobrevibacter, Methanomassiliicoccus* such as the species *Acetobacterium carbinolicum (ATCC BAA-990, DSM 2925), Acetobacterium malicum (DSM 4132), Acetobacterium wieringae (ATCC 43740, DSM 1911, JCM 2380), Blautia hydrogenotrophica* (DSM 10507, JCM 14656), *Blautia producta* (ATCC 27340, DSM 2950, JCM 1471), *Clostridium aceticum (ATCC 35044, DSM 1496, JCM 15732), Clostridium glycolicum (ATCC 14880, DSM 1288, JCM 1401), Clostridium magnum (ATCC 49199, DSM 2767), Clostridium mayombe (ATCC 51428, DSM 2767), Methanobrevibacter smithii* (ATCC 35061, DSM 861, JCM 328), *Candidatus Methanomassiliicoccus intestinalis, Eubacterium hallii (ATCC 27751, DSM 3353, JCM 31263), Eubacterium limosum (A TCC 8486, DSM 20543, JCM 6421), and Eubacterium ramulus (ATCC 29099, DSM 15684, JCM).*

Optionally, group (A9) comprises bacteria strains consuming sugars, fibers, formate and hydrogen, producing acetate and optionally butyrate. Such bacteria strains are known and include bacteria of the genus *Blautia* and archaea of the genera *Methanobrevibacter, Methanomassiliicoccus* such as the species *Blautia hydrogenotrophica* (DSM 10507, JCM 14656), *Blautia producta* (ATCC 27340, DSM 2950, JCM 1471), *Methanobrevibacter smithii* (ATCC 35061, DSM 861, JCM 328), *Candidatus Methanomassiliicoccus intestinalis.* Such bacteria strains further include bacteria of the genera *Acetobacterium, Clostridium, Moorella* and *Sporomusa,* such as the species *Acetobacterium carbinolicum* (ATCC BAA-990, DSM 2925), *Acetobacterium malicum* (DSM 4132), *Acetobacterium wieringae* (ATCC 43740, DSM 1911, JCM 2380), *Clostridium aceticum* (ATCC 35044, DSM 1496, JCM 15732), *Clostridium glycolicum* (ATCC 14880, DSM 1288, JCM 1401), *Clostridium magnum* (ATCC 49199, DSM 2767), *Clostridium mayombe* (ATCC 51428, DSM 2767).

**Further Groups** It is understood that additional bacteria functional groups (A10) to (A**), in particular (A10), (A11), (A12), (A13), (A14) and/or (A15), may also be present in the compositions described herein. Such groups may further improve the use of the compositions described herein. They may be added to the compositions in the amounts given above.

As an exemplary aspect, group (A10) may be mentioned:
**Group (A10)** comprises bacteria strains consuming sugars, fibers, and resistant starch, and producing succinate. In one embodiment, group (A10) is selected to cover bacteria producing succinate as a main metabolite. In one further embodiment, group (A10) is selected to cover bacteria producing succinate as a metabolite along with other metabolites, such as acetate and propionate.

Such bacteria strains are known and include bacteria of the genera *Alistipes, Bacteroides, Blautia, Barnesiella, Clostridium, Ruminococcus* and *Prevotella,* such as *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii.*

Optionally, the bacteria strains are selected from the genera *Alistipes, Bacteroides, Blautia, Clostridium, Ruminococcus* and *Prevotella,* such as the species *Bacteroides faecis* (DSM 24798, JCM 16478), *Bacteroides fragilis* (ATCC 25285, DSM 2151, JCM 11019), *Bacteroides ovatus* (ATCC 8483, DSM 1896, JCM 5824), *Bacteroides plebeius* (DSM 17135, JCM 12973), *Bacteroides uniformis* (ATCC 8492, DSM 6597, JCM 5828), *Bacteroides thetoiotoomicron* (ATCC 29148, DSM 2079, JCM 5827), *Bacteroides vulgatus* (ATCC 8482, DSM 1447, JCM 5826), *Bacteroides xylanisolvens* (DSM 18836, JCM 15633), *Blautia*/*Clostridium coccoides* (ATCC 29236, DSM 935, JCM 1395), *Blautia luti* (DSM 14534, JCM 17040), *Blautia wexlerae* (ATCC BAA-1564, DSM 19850, JCM 17041), *Clostridium butyricum* (ATCC 19398, DSM 10702, JCM 1391), *Clostridium bartlettii* (ATCC BAA-827, DSM 16795), *Ruminococcus callidus* (ATCC 27760), *Ruminococcus flavefaciens* (DSM 25089), *Prevotella copri* (DSM 18205, JCM 13464), *Prevotella stercorea* (DSM 18206, JCM 13469), *Alistipes finegoldii* (DSM 1724, JCM 16770), *Alistipes onderdonkii* (ATCC BAA-1178, DSM 19147, JCM 16771), and *Alistipes shahii* (ATCC BAA-1179, DSM 19121, JCM 16773).

In a preferred aspect, group (A10) is selected from bacteria of the genera *Alistipes, Bacteroides, Barnesiella, Ruminococcus* and *Prevotella,* such as the species *Bacteroides faecis* (DSM 24798, JCM 16478), *Bacteroides fragilis* (ATCC 25285, DSM 2151, JCM 11019), *Bacteroides ovatus* (ATCC 8483, DSM 1896, JCM 5824), *Bacteroides plebeius* (DSM 17135, JCM 12973), *Bacteroides uniformis* (ATCC 8492, DSM 6597, JCM 5828), *Bacteroides thetoiotoomicron* (ATCC 29148, DSM 2079, JCM 5827), *Bacteroides vulgatus* (ATCC 8482, DSM 1447, JCM 5826), *Bacteroides xylanisolvens* (DSM 18836, JCM 15633), *Barnesiella intestinihominis* (DSM 21032, JCM 15079), *Barnesiella viscericola* (DSM 18177, JCM 13660) *Ruminococcus callidus* (ATCC 27760), *Ruminococcus flavefaciens* (DSM 25089), *Prevotella copri* (DSM 18205, JCM 13464), *Prevotella stercorea* (DSM 18206, JCM 13469), *Alistipes finegoldii* (DSM 1724, JCM 16770), *Alistipes onderdonkii* (ATCC BAA-1178, DSM 19147, JCM 16771), and *Alistipes shahii* (ATCC BAA-1179, DSM 19121, JCM 16773).

**Group (A11)** comprises bacteria strains consuming proteins and producing acetate or butyrate. Such bacteria strains are known and include bacteria of the genera *Clostridium, Coprococcus, Eubacterium, Flavonifractor and Flintibacter,* such as the species *Clostridium butyricum (ATCC 19398, DSM 10702, JCM 1391), Coprococcus eutactus (ATCC 27759), Eubacterium hallii (ATCC 27751, DSM* 3353, *JCM* 31263), *Flavonifractor plautii (ATCC 29863, DSM 4000) and Flintibacter butyricum (DSM 27579).*

**Group (A12)** comprises bacteria strains consuming proteins, fibers, starches or sugars and producing biogenic amines such as γ-aminobutyric acid (GABA), cadaverine, dopamine, histamine, putrescine, serotonin, spermidine and/or tryptamine. Such bacteria strains are known and include bacteria of the genera *Bacteroides, Barnesiella, Bifidobacterium, Clostridium* (only tryptamine producers), *Enterococcus, Faecalibacterium, Lactobacillus* and *Ruminococcus* (only tryptamine producers), such as the species *Bacteroides caccae* (DSM 19024, ATCC 43185, JCM 9498), *Bacteroides faecis* (DSM 24798, JCM 16478), *Bacteroides fragilis* (DSM 2151, ATCC 25285, JCM 11019), *Bacteroides massiliensis* (DSM17679), *Bacteroides ovatus* (DSM 1896, ATCC 8483, JCM 5824), *Bacteroides uniformis* (DSM 6597, ATCC 8492, JCM 5828), *Bacteroides vulgatus* (DSM 1447, ATCC 8482), *Barnesiella intestinihominis* (DSM21032), *Bifidobacterium adolescentis* (DSM 20083, ATCC 15703) and *Lactobacillus plantarum* (DSM 2601, ATCC 10241) as GABA producers, *Clostridium sporogenes* (ATCC 15579), *Lactobacillus bulgaricus-*52 (NDRI) and *Ruminococcus gnavus* (ATCC 29149) as tryptamine producers, *Acidaminococcus intestini* (DSM 21505), *Bacteroides massiliensis* (DSM 17679), *Bacteroides stercoris* (ATCC 43183), *Enterococcus faecalis (ATCC 29212, DSM 2570), Enterococcus faecium (ATCC BAA-2317, DSM 7135)* and *Faecalibacterium prousnitzii* (DSM 17677) as putrescine producers, and *Clostridium bolteae* (ATCC BAA-613) as spermidine producers.

**Group (A13)** comprises bacteria strains consuming primary bile acids and producing secondary metabolites. Such bacteria strains are known and include bacteria of the genera *Anaerostipes, Blautia, Clostridium* and *Faecalibacterium,* such as the species *Anaerostipes coccoe (DSM14662), Blautia hydrogenotrophica (DSM 10507, JCM 14656), Clostridium bolteae (ATCC BAA-613), Clostridium scindens (DSM 5676, ATCC 35704), Clostridium symbiosum (ATCC14940) and Faecalibacterium prausnitzii (DSM 17677)*

**Group (A14)** comprises bacteria strains producing vitamins such as cobalamin (B12), folate (B9) or riboflavin (B2). Such bacteria are known in the art and include bacteria of the genera *Bacteroides, Bifidobacterium, Blautia, Clostridium, Faecalibacterium, Lactobacillus, Prevotella* and *Ruminococcus,* such as the species *Bacteroides fragilis (DSM 2151, ATCC* 25285, *JCM 11019), Bifidobacterium adolescentis (DSM 20083, ATCC 15703), Bifidobacterium pseudocatenulatum (ATCC 27919, DSM 20438, JCM 1200), Blautia hydrogenotrophica (DSM 10507, JCM 14656), Clostridium bolteae (ATCC BAA-613), Faecalibacterium prausnitzii (DSM 17677), Lactobacillus plantarum (DSM 2601, ATCC 10241), Prevotella copri (DSM 18205, JCM 13464) and Ruminococcus lactaris (ATCC 29176)*

**Group (A15)** comprises bacteria strains consuming mucus. Such bacteria are known in the art and include bacteria of the genera *Akkermansia, Bacteroides, Bifidobacterium* and *Ruminococcus;* such as the species *Akkermansia muciniphila (ATCC BAA-835), Bacteroides fragilis (DSM 2151, ATCC 25285, JCM 11019), Bacteroides thetoiotoomicron (ATCC 29148, DSM 2079, JCM 5827), Bifidobacterium bifidum (ATCC 29521, DSM 20456, JCM 1255), Ruminococcus gnavus (ATCC 29149, ATCC 35913, JCM 6515) and Ruminococcus torques (ATCC 27756).*

The bacteria strains as defined herein are in each case identified through classification of the full 16S rRNA gene with assignment for the different taxonomic levels Phylum: 75%, Class: 78.5%, Order: 82%, Family: 86.5%, Genus: 94.5%, Species: 98.65% of sequence similarity, preferably of the whole 16S. Such assignment may be achieved by using SILVA Software (SSURef NR99 128 SILVA) and using the HITdb (Ritari et al., 2015).

Any of the above bacterial strains can be combined together in a consortium as long as all functional group A1 to A9 are represented, optionally with additional groups A10, A11, A12, A13, A14 and/or A15. Such consortium can comprise one or more bacterial strain per functional groups.

Preferably, all of the functional groups A1 to A**, more particularly A1 to A9, optionally with additional groups A10, A11, A12, A13, A14 and/or A15, are represented in a preferred consortium of the present invention. As discussed above, all bacterial strains are defined by their functions. Such functions may be accomplished by one or more than one bacterial strain. Accordingly, each functional group comprises one or more, preferably one, bacterial strains. Alternatively, one bacterium can be able to perform a plurality of functions, i.e. can belong to one or more functional group.

In some embodiments, the consortium comprises at least one bacterial strain in each of the A1, A2, A3, A4, A5, A6, A7, A8 and A9 functional groups. Optionally, it further comprises a bacterial strain of functional group A10 and/or a bacterial strain of functional group A11, A12, A13, A14 and/or A15. Optionally, the consortium may comprise a bacterial strain that belongs to more than one functional group of the A1, A2, A3, A4, A5, A6, A7, A8 and A9 functional groups. Then, a particular bacterial strain can belong to 2, 3 or 4 functional groups. In one embodiment, the consortium comprises a bacterial strain that belongs to both of the functional groups A6 and A9, i.e. such bacterial strain being capable of performing features of functional groups A6 and A9. In another embodiment, the consortium comprises a bacterial strain that belongs to both of the functional groups A4 and A7, i.e. such bacterial strain being capable of performing features of functional groups A4 and A7.

Then, if each bacteria strain of the consortium belongs to a different functional group, the consortium can be composed by at least 9 or 10 bacteria.

Alternatively, if a particular bacterial strain belongs to at least two functional groups (e.g. A6 and A9 or A4 and A7), then the consortium may comprise less than 9 or 10 bacterial strains, preferably 8, 7, 6, 5, 4 or 3 bacterial strains.

In addition, the consortium may also comprise more than one bacterial strain for one functional group, the consortium is composed of more than 9 or 10 bacterial strains, preferably 11, 12, 13, 14, 15, 16, 17, 18, 19, 20, 25, 30, 35, 40, 45 or 50 bacteria.

Then, the composition according to the invention comprises functional groups A1 to A9, optionally optionally in combination with (A10), (A11), (A12), (A13), (A14) and/or (A15) or subsets thereof, wherein functional groups A1 to A15, are:
(A1) Sugars, fibers and resistant starch degraders and formate and acetate producers,
(A2) Sugar and starch degrading-, acetate-consuming and butyrate- and formate- producers,
(A3) Oxygen-reducing, sugar consuming, and lactate- producers,
(A4) Starch-reducing, sugar and carbon dioxide consumers, and lactate-, acetate- and formate-producers,
(A5) Protein- and lactate-utilizing, and propionate- and acetate- producers,
(A6) Starch- and lactate-utilizing and butyrate-, acetate and hydrogen producers,
(A7) Sugar and starch-degrading formate-, lactate- and lactate-producers,
(A8) Succinate-utilizing, and propionate- and acetate producers,
(A9) Hydrogen- and formate-utilizing, sugars and fiber consuming and acetate-producers,
(A10) is an additional/optional functional group of succinate producers,
(A11) is an additional/optional functional group of protein- utilizer and producers of acetate and/or butyrate.
(A12) is an additional/optional functional group of proteins, fibers, starches or sugars consumers and biogenic amines producers such as γ-aminobutyric acid (GABA), cadaverine, dopamine, histamine, putrescine, serotonin, spermidine and/or tryptamine producers.
(A13) is an additional/optional functional group of primary bile acids consumers and secondary metabolites producers.
(A14) is an additional/optional functional group of vitamins producers such as cobalamin (B12), folate (B9) or riboflavin (B2).
(A15) is an additional/optional functional group of mucus degraders.

Preferably, the composition comprises:
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars and oxygen, and producing lactate (A3);
- at least one bacterial strain consuming sugars, starch, and carbon dioxide, and producing lactate, formate and acetate (A4),
- at least one bacterial strain consuming lactate or proteins, and producing propionate and acetate (A5);
- at least one bacterial strain consuming lactate and starch, and producing acetate, butyrate and hydrogen (A6);
- at least one bacterial strain consuming sugar, starch, and formate and producing lactate, formate and acetate (A7);
- at least one bacterial strain consuming succinate, and producing propionate and acetate (A8); and
- at least one bacterial strain consuming sugars, fibers, formate and hydrogen, and producing acetate and optionally butyrate (A9); and
- Optionally:
   - at least one bacterial strain consuming sugars, fibers, and resistant starch, and producing succinate (A10);
   - at least one bacterial strain consuming proteins and producing acetate and butyrate (A11);
   - at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
   - at least one bacterial strain consuming primary bile acids and producing secondary metabolites (A13);
   - at least one bacterial strain producing vitamins such as cobalamin (B12), folate (B9) or riboflavin (B2), (A14); and/or
   - at least one bacterial strain consuming mucus (A15).

In a first particular aspect, the composition comprises:
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars and oxygen, and producing lactate (A3);
- -at least one bacterial strain consuming sugars, starch, and carbon dioxide, and producing lactate, formate and acetate (A4),
- at least one bacterial strain consuming lactate or proteins, and producing propionate and acetate (A5);
- at least one bacterial strain consuming lactate, fibers, formate, hydrogen and starch, and producing acetate, butyrate and hydrogen ((A6) and (A9));
- at least one bacterial strain consuming sugar, starch, and formate and producing lactate, formate and acetate (A7);
- at least one bacterial strain consuming succinate, and producing propionate and acetate (A8); and optionally:
- at least one bacterial strain consuming sugars, fibers, and resistant starch, and producing succinate (A10);
- at least one bacterial strain consuming proteins and producing acetate and butyrate (A11);
- at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
- at least one bacterial strain consuming primary bile acids and producing secondary metabolites (A13);
- at least one bacterial strain producing vitamins such as cobalamin (B12), folate (B9) or riboflavin (B2), (A14); and/or
- at least one bacterial strain consuming mucus (A15).

In a second particular aspect, the composition comprises:
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars and oxygen, and producing lactate (A3);
- at least one bacterial strain consuming sugars, starch, formate and carbon dioxide, and producing lactate, formate and acetate ((A4) and (A7)),
- at least one bacterial strain consuming lactate or proteins, and producing propionate and acetate (A5);
- at least one bacterial strain consuming lactate and starch, and producing acetate, butyrate and hydrogen (A6);
- at least one bacterial strain consuming succinate, and producing propionate and acetate (A8); and
- at least one bacterial strain consuming sugars, fibers, formate and hydrogen, and producing acetate and optionally butyrate (A9);
optionally:
-- at least one bacterial strain consuming sugars, fibers, and resistant starch, and producing succinate (A10);
-- at least one bacterial strain consuming proteins and producing acetate and butyrate (A11);
-- at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
-- at least one bacterial strain consuming primary bile acids and producing secondary metabolites (A13);
-- at least one bacterial strain producing vitamins such as cobalamin (B12), folate (B9) or riboflavin (B2), (A14); and/or
-- at least one bacterial strain consuming mucus (A15).

In a third particular aspect, the composition comprises:
- at least one bacterial strain consuming sugars, fibers, and resistant starch, and producing formate and acetate (A1);
   --at least one bacterial strain consuming sugars, starch and acetate, and producing formate and butyrate (A2);
- at least one bacterial strain consuming sugars and oxygen, and producing lactate (A3);
- at least one bacterial strain consuming sugars, starch, formate and carbon dioxide, and producing lactate, formate and acetate ((A4) and (A7)),
- at least one bacterial strain consuming lactate or proteins, and producing propionate and acetate (A5);
- at least one bacterial strain consuming lactate, fibers, formate and hydrogen and starch, and producing acetate, butyrate and hydrogen (A6) and (A9);
- at least one bacterial strain consuming succinate, and producing propionate and acetate (A8); and optionally:
- at least one bacterial strain consuming sugars, fibers, and resistant starch, and producing succinate (A10);
- at least one bacterial strain consuming proteins and producing acetate and butyrate (A11);
- at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
- at least one bacterial strain consuming primary bile acids and producing secondary metabolites (A13);
- at least one bacterial strain producing vitamins such as cobalamin (B12), folate (B9) or riboflavin (B2), (A14); and/or
- at least one bacterial strain consuming mucus (A15).

Preferably, such composition comprises:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera *Lactobacillus, Streptococcus, Escherichia, Lactococcus* and *Enterococcus* (A3);
- at least one bacterial strain of the genus *Bifidobacterium* or *Roseburia* (A4), optionally of the genus *Bifidobacterium;*
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain selected from the genera *Anaerostipes, Clostridium* and *Eubacterium* (A6),
- at least one bacterial strain of the genus *Collinsella* or *Roseburia* (A7), optionally of the genus *Collinsella;*
- at least one bacterial strain selected from the genera *Phascolarctobacterium, Flavonifractor* and *Dialister* (A8), optionally selected from the genera *Phascolarctobacterium* and *Dialister;* and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa;*
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

In a first particular aspect, the composition comprises:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera *Lactobacillus, Streptococcus, Escherichia, Lactococcus* and *Enterococcus* (A3);
- at least one bacterial strain of the genus *Bifidobacterium* or *Roseburia* (A4), optionally of the genus *Bifidobacterium;*
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain of Eubacterium (A6) and (A9),
- at least one bacterial strain of the genus *Collinsella* or *Roseburia* (A7), optionally of the genus *Collinsella;*
- at least one bacterial strain selected from the genera *Phascolarctobacterium, Flavonifractor* and *Dialister* (A8), optionally selected from the genera *Phascolarctobacterium* and *Dialister;* and
- optionally at least one bacterial strain selected from the genera *Alistipes, Bacteroides, Blautia, Barnesiella, Clostridium, Ruminococcus* and *Prevotella,* optionally *Alistipes, Bacteroides, Blautia, Clostridium, Ruminococcus* and *Prevotella,* preferably *Alistipes, Bacteroides, Barnesiella, Ruminococcus* and *Prevotella* (A10);
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium, Enterococcus, Faecalibacterium, Lactobacillus* and *Ruminococcus* (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

In a second particular aspect, the composition comprises:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera *Lactobacillus, Streptococcus, Escherichia, Lactococcus* and *Enterococcus* (A3);
- at least one bacterial strain of the genus *Roseburia* (A4) and (A7);
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain selected from the genera *Anaerostipes, Clostridium* and *Eubacterium* (A6),
- at least one bacterial strain selected from the genera *Phascolarctobacterium, Flavonifractor* and *Dialister* (A8), optionally selected from the genera *Phascolarctobacterium* and *Dialister;* and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa;*
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

In a third particular aspect, the composition comprises:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera Lactobacillus, Streptococcus, Escherichia, Lactococcus and Enterococcus (A3);
- at least one bacterial strain of the genus *Roseburia* (A4) and (A7);
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain of *Eubacterium* (A6) and (A9),
- at least one bacterial strain selected from the genera *Phascolarctobacterium, Flavonifractor* and *Dialister* (A8), optionally selected from the genera *Phascolarctobacterium* and *Dialister;* and
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Bloutic, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Even more specifically, the composition comprises:
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Lactobacillus rhamnosus, Streptococcus salivarius, Escherichia coli, Lactococcus lactis, Enterococcus faecalis, Enterococcus caccae* and any combination thereof (A3), optionally selected from *Lactobacillus rhamnosus, Streptococcus salivarius, Escherichia coli, Lactococcus lactis, Enterococcus caccae* and any combination thereof (A3);
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburic hominis* and any combination thereof (A4), optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum* and any combination thereof (A4);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Anaerostipes coccoe, Clostridium indolis, Eubacterium hallii, Eubacterium limosum, Eubacterium ramulus* and any combination thereof (A6);
- *Collinsella aerofaciens, Collinsella intestinalis, Collinsella stercoris, Roseburia hominis* and any combination thereof (A7), optionally selected from *Collinsella aerofaciens, Collinsella intestinalis, Collinsella stercoris* and any combination thereof (A7);
- *Phascolarctobacterium faecium, Dialister succinatiphilus, Flavonifractor plautii, Dialister propionifaciens* and any combination thereof (A8), optionally selected from *Phascolarctobacterium faecium, Dialister succinatiphilus, Dialister propionifaciens* and any combination thereof (A8); and
- *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii, Candidatus Methanomassiliicoccus intestinalis* and any combination thereof (A9), optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii, Candidatus Methanomassiliicoccus intestinalis* and any combination thereof (A9);
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides coccoe, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes coccoe, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Blautia hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a particular embodiment, the present invention relates to a composition comprising a consortium as disclosed herein which comprises *Enterococcus faecalis,* belonging to the functional group A3.

In one aspect, the present invention relates to a composition that comprises a consortium comprising *Enterococcus faecalis* (A3) and
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars, starch, and carbon dioxide, and producing lactate, formate and acetate (A4),
- at least one bacterial strain consuming lactate or proteins, and producing propionate and acetate (A5);
- at least one bacterial strain consuming lactate and starch, and producing acetate, butyrate and hydrogen (A6);
- at least one bacterial strain consuming sugar, starch, formate and producing lactate, formate and acetate (A7);
- at least one bacterial strain consuming succinate, and producing propionate and acetate (A8); and
- at least one bacterial strain consuming sugars, fibers, formate and hydrogen, and producing acetate and optionally butyrate (A9);
- optionally at least one bacterial strain consuming sugars, fibers, and resistant starch, and producing succinate (A10);
- optionally at least one bacterial strain consuming proteins and producing acetate and butyrate (A11);
- optionally at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
- optionally at least one bacterial strain consuming primary bile acids and producing secondary metabolites (A13);
- optionally at least one bacterial strain producing vitamins such as cobalamin (B12), folate (B9) or riboflavin (B2), (A14); and/or
- optionally at least one bacterial strain consuming mucus (A15).

Optionally, the composition may comprise
- at least one bacterial strain consuming lactate, fibers, formate and hydrogen and starch, and producing acetate, butyrate and hydrogen (A6) and (A9); and/or
- at least one bacterial strain consuming sugars, starch, formate and carbon dioxide, and producing lactate, formate and acetate (A4) and (A7).

More specifically, the composition may comprise:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- *Enterococcus faecalis* (A3);
- at least one bacterial strain of the genus *Bifidobacterium* or *Roseburia* (A4), optionally of the genus *Bifidobacterium;*
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain selected from the genera *Anaerostipes, Clostridium* and *Eubacterium* (A6),
- at least one bacterial strain of the genus *Collinsella* or *Roseburia* (A7), optionally of the genus *Collinsella;*
- at least one bacterial strain selected from the genera *Phascolarctobacterium, Flavonifractor* and *Dialister* (A8), optionally selected from the genera *Phascolarctobacterium* and *Dialister;* and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa;*
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Optionally, the composition may comprise at least one bacterial strain of *Eubacterium* (A6) and (A9), and/or at least one bacterial strain of the genus *Roseburia* (A4) and (A7).

Still more specifically, the present invention relates to a composition comprising a consortium comprising *Enterococcus faecalis* (A3) and
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburic hominis* and any combination thereof (A4), optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum* and any combination thereof (A4);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Anaerostipes caccae, Clostridium indolis, Eubacterium hallii, Eubacterium limosum, Eubacterium ramulus* and any combination thereof (A6);
- *Collinsella aerofaciens, Collinsella intestinalis, Collinsella stercoris, Roseburia hominis* and any combination thereof (A7), optionally selected from *Collinsella aerofaciens, Collinsella intestinalis, Collinsella stercoris* and any combination thereof (A7);
- *Phascolarctobacterium faecium, Dialister succinatiphilus, Flavonifractor plautii, Dialister propionifaciens* and any combination thereof (A8), optionally selected from *Phascolarctobacterium faecium, Dialister succinatiphilus, Dialister propionifaciens* and any combination thereof (A8); and
- *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii, Candidatus Methanomassiliicoccus intestinalis* and any combination thereof (A9), optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii, Candidatus Methanomassiliicoccus intestinalis* and any combination thereof (A9);
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides coccoe, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prousnitzii* as putrescine producers, and *Clostridium bolteae* as spermidine producers and any combination thereof (A12)
- optionally *Anaerostipes coccoe, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Blautia hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a very particular aspect, the composition comprises a consortium comprising *Eubacterium limosum* (A6) and (A9); and/or *Roseburia hominis* (A4) and (A7).

In a particular embodiment, the present invention relates to composition that comprises a consortium which comprises *Roseburia hominis,* belonging to the functional group A4 and A7.

In one aspect, the present invention relates to a composition comprising a consortium comprising *Roseburic hominis* (A4) and (A7) and:
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars and oxygen, and producing lactate (A3);
- at least one bacterial strain consuming lactate or proteins, and producing propionate and acetate (A5);
- at least one bacterial strain consuming lactate and starch, and producing acetate, butyrate and hydrogen (A6);
- at least one bacterial strain consuming succinate, and producing propionate and acetate (A8); and
   --at least one bacterial strain consuming sugars, fibers, formate and hydrogen, and producing acetate and optionally butyrate (A9);
   optionally at least one bacterial strain consuming sugars, fibers, and resistant starch, and producing succinate (A10); and
- optionally at least one bacterial strain consuming proteins and producing acetate and butyrate (A11);
- optionally at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
- optionally at least one bacterial strain consuming primary bile acids and producing secondary metabolites (A13);
- optionally at least one bacterial strain producing vitamins such as cobalamin (B12), folate (B9) or riboflavin (B2), (A14); and/or
- optionally at least one bacterial strain consuming mucus (A15).

Optionally, the consortium may comprise at least one bacterial strain consuming lactate, fibers, formate and hydrogen and starch, and producing acetate, butyrate and hydrogen (A6) and (A9).

More specifically, the composition may comprise a consortium comprising:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburic* and *Anaerostipes;*
- at least one bacterial strain selected from the genera *Lactobacillus, Streptococcus, Escherichia, Lactococcus and Enterococcus* (A3);
- *Roseburia hominis* (A4) and (A7);
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain selected from the genera *Anaerostipes, Clostridium* and *Eubacterium* (A6),
- at least one bacterial strain selected from the genera *Phascolarctobacterium, Flavonifractor* and *Dialister* (A8), optionally selected from the genera *Phascolarctobacterium* and *Dialister;* and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa;*
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).
- Optionally, the consortium may comprise at least one bacterial strain of *Eubacterium* (A6) and (A9). Still more specifically, the present invention relates to a consortium comprising *Roseburic hominis* (A4) and (A7); and
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Lactobacillus rhamnosus, Streptococcus salivarius, Escherichia coli, Lactococcus lactis, Enterococcus faecalis, Enterococcus caccae* and any combination thereof (A3), optionally selected from *Lactobacillus rhamnosus, Streptococcus salivarius, Escherichia coli, Lactococcus lactis, Enterococcus caccae* and any combination thereof (A3);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Anaerostipes caccae, Clostridium indolis, Eubacterium hallii, Eubacterium limosum, Eubacterium ramulus* and any combination thereof (A6);
- *Phascolarctobacterium faecium, Dialister succinatiphilus, Flavonifractor plautii, Dialister propionifaciens* and any combination thereof (A8), optionally selected from *Phascolarctobacterium faecium, Dialister succinatiphilus, Dialister propionifaciens* and any combination thereof (A8); and
- *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii, Candidatus Methanomassiliicoccus intestinalis* and any combination thereof (A9), optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii, Candidatus Methanomassiliicoccus intestinalis* and any combination thereof (A9);
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides caccae, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes caccae, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Blautia hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a very particular aspect, the consortium comprises *Eubacterium limosum* (A6) and (A9) and/or *Enterococcus faecalis* (A3).

In a particular embodiment, the present invention relates to a consortium as disclosed herein which comprises *Eubacterium limosum,* belonging to the functional groups A6 and A9.

In a particular aspect, when the consortium comprises *Eubacterium limosum,* belonging to the functional group A6 and A9, it does not comprise any bacterium or archaea from the genus Acetobacterium, Blautia, Moorella, Methanobrevibacter, Methanomassiliicoccus and Sporomusa (A9).

In one aspect, the present invention relates to a consortium comprising *Eubacterium limosum* ((A6) and (A9)) and:
- at least one bacterial strain consuming sugars, fibers, and resistant starch, and producing formate and acetate (A1);
- at least one bacterial strain consuming sugars, starch and acetate, and producing formate and butyrate (A2);
- at least one bacterial strain consuming sugars and oxygen, and producing lactate (A3);
- at least one bacterial strain consuming sugars, starch, and carbon dioxide, and producing lactate, formate and acetate (A4),
- at least one bacterial strain consuming lactate or proteins, and producing propionate and acetate (A5);
- at least one bacterial strain consuming sugar, starch, formate and producing lactate, formate and acetate (A7);
- at least one bacterial strain consuming succinate, and producing propionate and acetate (A8); and
- optionally at least one bacterial strain consuming sugars, fibers, and resistant starch, and producing succinate (A10); and
- optionally at least one bacterial strain consuming proteins and producing acetate and butyrate (A11);
- optionally at least one bacterial strain consuming proteins, fibers, starches or sugars and producing biogenic amines such as γ-aminobutyric acid (GABA), cadaverine, dopamine, histamine, putrescine, serotonin, spermidine and/or tryptamine (A12);
- optionally at least one bacterial strain consuming primary bile acids and producing secondary metabolites (A13);
- optionally at least one bacterial strain producing vitamins such as cobalamin (B12), folate (B9) or riboflavin (B2), (A14); and/or
- optionally at least one bacterial strain consuming mucus (A15).

Optionally, the consortium may comprise at least one bacterial strain consuming lactate, fibers, formate and hydrogen and starch, and producing acetate, butyrate and hydrogen (A6) and (A9).

More specifically, the consortium may comprise
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera Lactobacillus, Streptococcus, Escherichia, Lactococcus and Enterococcus (A3);
- at least one bacterial strain of the genus *Bifidobacterium* or *Roseburia* (A4), optionally of the genus *Bifidobacterium;*
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- *Eubacterium limosum* (A6) and (A9);
- at least one bacterial strain of the genus *Collinsella* or *Roseburia* (A7), optionally of the genus *Collinsella;*
- at least one bacterial strain selected from the genera *Phascolarctobacterium, Flavonifractor* and *Dialister* (A8), optionally selected from the genera *Phascolarctobacterium* and *Dialister;* and
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Optionally, the consortium may comprise at least one bacterial strain of Roseburia (A4) and (A7).

Still more specifically, the present invention relates to a consortium comprising *Eubacterium limosum* (A6) and (A9); and
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Lactobacillus rhamnosus, Streptococcus salivarius, Escherichia coli, Lactococcus lactis, Enterococcus faecalis, Enterococcus caccae* and any combination thereof (A3), optionally selected from *Lactobacillus rhamnosus, Streptococcus salivarius, Escherichia coli, Lactococcus lactis, Enterococcus caccae* and any combination thereof (A3);
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburic hominis* and any combination thereof (A4), optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum* and any combination thereof (A4);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Eubacterium limosum* (A6) and (A9);
- *Collinsella aerofaciens, Collinsella intestinalis, Collinsella stercoris, Roseburia hominis* and any combination thereof (A7), optionally selected from *Collinsella aerofaciens, Collinsella intestinalis, Collinsella stercoris* and any combination thereof (A7);
- *Phascolarctobacterium faecium, Dialister succinatiphilus, Flavonifractor plautii, Dialister propionifaciens* and any combination thereof (A8), optionally selected from *Phascolarctobacterium faecium, Dialister succinatiphilus, Dialister propionifaciens* and any combination thereof (A8); and
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides coccoe, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes coccoe, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Blautia hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a very particular aspect, the consortium comprises *Roseburia hominis* (A4) and (A7) and *Enterococcus faecalis* (A3) and:
- at least one bacterial strain selected from the genera *Ruminococcus, Dorea, Clostridium* and *Eubacterium* (A1), optionally selected from the genera *Ruminococcus, Dorea* and *Eubacterium;*
- at least one bacterial strain selected from the genera *Faecalibacterium, Roseburia, Anaerostipes* and *Eubacterium* (A2), optionally selected from the genera *Faecalibacterium, Roseburia* and *Anaerostipes;*
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera;*
- at least one bacterial strain selected from the genera *Anaerostipes, Clostridium* and *Eubacterium* (A6),
- at least one bacterial strain selected from the genera *Phascolarctobacterium, Flavonifractor* and *Dialister* (A8), optionally selected from the genera *Phascolarctobacterium* and *Dialister;* and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa;*
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella;*
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Still more specifically, the present invention relates to a consortium comprising *Roseburia hominis* ((A4) and (A7)) and *Enterococcus faecalis* (A3) and:
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus chomponellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Anaerostipes caccae, Clostridium indolis, Eubacterium hallii, Eubacterium limosum, Eubacterium ramulus* and any combination thereof (A6);
- *Phascolarctobacterium faecium, Dialister succinatiphilus, Flavonifractor plautii, Dialister propionifaciens* and any combination thereof (A8), optionally selected from *Phascolarctobacterium faecium, Dialister succinatiphilus, Dialister propionifaciens* and any combination thereof (A8); and
- *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii, Candidatus Methanomassiliicoccus intestinalis* and any combination thereof (A9), optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii, Candidatus Methanomassiliicoccus intestinalis* and any combination thereof (A9);
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides caccae, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes caccae, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Bloutic hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a particular embodiment, the present invention relates to a consortium as disclosed herein which comprises *Flavonifractor plautii,* belonging to the functional group A8.

In one aspect, the present invention relates to a consortium comprising *Flavonifractor plautii* (A8) and:
- at least one bacterial strain selected from the genera *Ruminococcus, Dorea, Clostridium* and *Eubacterium* (A1), optionally selected from the genera *Ruminococcus, Dorea* and *Eubacterium*;
- at least one bacterial strain selected from the genera *Faecalibacterium, Roseburia, Anaerostipes* and *Eubacterium* (A2), optionally selected from the genera *Faecalibacterium, Roseburia* and *Anaerostipes*;
- at least one bacterial strain selected from the genera Lactobacillus, Streptococcus, Escherichia, Lactococcus and Enterococcus (A3);
- at least one bacterial strain of the genus *Bifidobacterium* or *Roseburia* (A4), optionally of the genus *Bifidobacterium*;
- at least one bacterial strain selected from the genera *Clostridium, Propionibacterium, Veillonella, Coprococcus* and *Megasphaera* (A5), optionally selected from the genera *Clostridium, Propionibacterium, Veillonella* and *Megasphaera*;
- at least one bacterial strain selected from the genera *Anaerostipes, Clostridium* and *Eubacterium* (A6),
- at least one bacterial strain of the genus *Collinsella* or *Roseburia* (A7), optionally of the genus *Collinsella*; and
- at least one bacterial strain selected from the genera *Acetobacterium, Blautia, Clostridium, Eubacterium, Moorella, Methanobrevibacter, Methanomassiliicoccus* and *Sporomusa* (A9), optionally selected from the genera *Acetobacterium, Blautia, Clostridium, Moorella, Methanobrevibacter, Methanomassiliicoccus* and *Sporomusa*;
- optionally at least one bacterial strain selected from the genera *Alistipes, Bacteroides, Blautia, Barnesiella, Clostridium, Ruminococcus* and *Prevotella* (A10), optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella*;
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Blautia, Clostridium* and *Faecalibacterium* (A13)
- optionally at least one bacterial strain selected from the genera *Bacteroides, Bifidobacterium, Blautia, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Still more specifically, the present invention relates to a consortium comprising *Flavonifractor plautii* (A8) and:
- *Ruminococcus bromii, Ruminococcus lactaris, Ruminococcus chomponellensis, Ruminococcus callidus, Ruminococcus gnavus, Ruminococcus obeum, Clostridium scindens, Dorea longicatena, Dorea formicigenerans, Eubacterium eligens* and any combination thereof (A1), optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1);
- *Faecalibacterium prausnitzii, Anaerostipes hadrus, Roseburia intestinalis Eubacterium ramulus, Eubacterium rectale* and any combination thereof (A2), optionally selected from *Faecalibacterium prausnitzii, Anaerostipes hadrus, Roseburia intestinalis* and any combination thereof (A2);
- *Lactobacillus rhamnosus, Streptococcus salivarius, Escherichia coli, Lactococcus lactis, Enterococcus faecalis, Enterococcus caccae* and any combination thereof (A3), optionally selected from *Lactobacillus rhamnosus, Streptococcus salivarius, Escherichia coli, Lactococcus lactis, Enterococcus caccae* and any combination thereof (A3);
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburic hominis* and any combination thereof (A4), optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum* and any combination thereof (A4);
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5), optionally selected from *Clostridium aminovalericum, Clostridium celatum, Clostridium lactatifermentans, Clostridium neopropionicum, Clostridium propionicum, Megasphaera elsdenii, Veillonella montpellierensis, Veillonella ratti* and any combination thereof (A5);
- *Anaerostipes caccae, Clostridium indolis, Eubacterium hallii, Eubacterium limosum, Eubacterium ramulus* and any combination thereof (A6);
- *Collinsella aerofaciens, Collinsella intestinalis, Collinsella stercoris, Roseburia hominis* and any combination thereof (A7), optionally selected from *Collinsella aerofaciens, Collinsella intestinalis, Collinsella stercoris* and any combination thereof (A7); and
- *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii, Candidatus Methanomassiliicoccus intestinalis* and any combination thereof (A9), optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii, Candidatus Methanomassiliicoccus intestinalis* and any combination thereof (A9);
- optionally *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10), optionally from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii* and any combination thereof (A10), preferably from *Bacteroides faecis, Bacteroides fragilis, Bacteroides ovatus, Bacteroides plebeius, Bacteroides uniformis, Bacteroides thetoiotoomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10);
- optionally *Clostridium butyricum, Coprococcus eutactus, Eubacterium hallii, Flavonifractor plautii and Flintibacter butyricum* and any combination thereof (A11);
- optionally *Bacteroides caccae, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-*52 and *Ruminococcus gnavus* as tryptamine producers, *Acidaminococcus intestini, Bacteroides mossiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes caccae, Blautia hydrogenotrophica, Clostridium bolteae, Clostridium scindens, Clostridium symbiosum* and *Faecalibacterium prousnitzii* and any combination thereof (A13)
- optionally *Bacteroides fragilis, Bifidobacterium adolescentis, Bifidobacterium pseudocatenulatum, Bloutic hydrogenotrophica, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetoiotoomicron, Bifidobacterium bifidum, Ruminococcus gnavus and Ruminococcus torques* and any combination thereof (A15).

In a particular embodiment, the consortium comprises or essentially consists of:
- *Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6), *Collinsella aerofaciens* (A7), *Phascolarctobacterium faecium* (A8), and *Blautia hydrogenotrophica* (A9) and optionally *Bacteroides xylanisolvens* (A10).

Alternatively, the consortium comprises or essentially consists of:
- *Ruminococcus bromii* (A1), *Faecalibacterium prousnitzii* (A2), *Lactobacillus rhamnosus* (A3), *Bifidobacterium adolescentis* (A4), *Anaerotignum lactatifermentans* (A5), *Eubacterium limosum* (A6 and A9), *Collinsella aerofaciens* (A7) and *Phascolarctobacterium faecium* (A8) and optionally *Bacteroides xylanisolvens* (A10);

Alternatively, the consortium comprises or essentially consists of:
- *Eubacterium eligens* (A1), *Roseburia intestinalis* (A2), *Enterococcus faecalis* (A3), *Roseburia* hominis (A4 and A7), *Coprococcus catus* (A5), *Eubacterium hallii* (A6), *Flavonifractor plautii* (A8), *Eubacterium limosum* (A9) and optionally *Bacteroides xylanisolvens* (A10).

Alternatively, the consortium comprises or essentially consists of:
- *Eubacterium eligens* (A1), *Roseburia intestinalis* (A2), *Enterococcus faecalis* (A3), *Roseburia* hominis (A4 and A7), *Coprococcus catus* (A5), *Eubacterium limosum* (A6 and A9), and *Flavonifractor plautii* (A8),

In a particular aspect, the consortium is such that it does not comprise a bacterium from the genus Blautia, nor an archaea of the genus Methanobrevibacter or Methanomassiliicoccus, especially *Blautia hydrogenotrophica, Blautia producta, Methanobrevibacter smithii* and *Candidatus Methanomassiliicoccus intestinalis,* particularly when the consortium comprises Eubacterium limosum, particularly when the consortium comprises *Eubacterium limosum* such as to fulfils the metabolic function of functional group A9, preferably A9 and A6.

In a particular aspect, the consortium is such that it does not comprise a bacterium from the genus Blautia, *Acetobacterium, Clostridium, Moorella,* and Sporomusa, nor an archaea of the genus Methanobrevibacter or Methanomassiliicoccus, especially *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii* and *Candidatus Methanomassiliicoccus intestinalis,* particularly when the consortium comprises Eubacterium limosum, particularly when the consortium comprises *Eubacterium limosum* such as to fulfils the metabolic function of functional group A9, preferably A9 and A6.

In a particular aspect, preferably when the consortium comprises an Eubacterium, preferably *Eubacterium limosum,* the consortium is such that it does not comprise *Blautia hydrogenotrophica.*

In another particular aspect, the consortium is such that it does not comprise a bacterium from the genus Blautia, especially *Bloutic hydrogenotrophica* and/or *Bloutic producta,* particularly when the consortium comprises an Eubacterium, preferably *Eubacterium limosum.*

Additionally or alternatively, particularly when the consortium comprises an Eubacterium, preferably *Eubacterium limosum,* the consortium is such that it does not comprise:
- an archaea of the genus Methanobrevibacter or Methanomassiliicoccus, preferably *Methanobrevibacter smithii* and/or *Candidatus Methanomassiliicoccus intestinalis,*
- a bacterium of the genera *Acetobacterium,* preferably *Acetobacterium carbinolicum, Acetobacterium malicum* and/or *Acetobacterium wieringae,*
- a bacterium of the genera *Moorella* and/or *Sporomusa;* and/or
   -- a bacterium selected from *Clostridium aceticum, Clostridium glycolicum, Clostridium magnum* and/or *Clostridium mayombe.*

Then, in one embodiment, the consortium comprises or essentially consists in *Eubacterium limosum* (A6 + A9) and:
- at least one bacterial strain selected from the genera *Ruminococcus, Dorea, Clostridium* and *Eubacterium* (A1);
- at least one bacterial strain selected from the genera *Faecalibacterium, Roseburia, Anaerostipes* and *Eubacterium* (A2);
- at least one bacterial strain selected from the genera Lactobacillus, Streptococcus, Escherichia, Lactococcus and Enterococcus (A3);
- at least one bacterial strain of the genus *Bifidobacterium* or *Roseburia* (A4);
- at least one bacterial strain selected from the genera *Clostridium, Propionibacterium, Veillonella, Coprococcus* and *Megasphaera* (A5);
- at least one bacterial strain of the genus *Collinsella* or *Roseburia* (A7);
- at least one bacterial strain selected from the genera *Phascolarctobacterium, Flavonifractor* and *Dialister* (A8); and
- optionally at least one bacterial strain selected from the genera *Alistipes, Bacteroides, Barnesiella, Clostridium, Ruminococcus* and *Prevotella* (A10);
- optionally at least one bacterial strain selected from the genera *Clostridium, Coprococcus, Eubacterium, Flavonifractor and Flintibacter* (A11);
- optionally at least one bacterial strain selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers) (A12);
- optionally at least one bacterial strain selected from the genera *Anaerostipes, Clostridium* and *Faecalibacterium* (A13)
- optionally at least one bacterial strain selected from the genera *Bacteroides, Bifidobacterium, Clostridium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

Particularly, the consortium comprises or essentially consists in *Eubacterium limosum* (A6 + A9) and:
- at least one bacterial strain selected from the genera *Ruminococcus, Dorea,* and *Eubacterium* (A1);
- at least one bacterial strain selected from the genera *Faecalibacterium, Roseburia, Anaerostipes* and *Eubacterium* (A2);
- at least one bacterial strain selected from the genera *Lactobacillus, Streptococcus, Escherichia, Lactococcus* and *Enterococcus* (A3);
- at least one bacterial strain of the genus *Bifidobacterium* or *Roseburia* (A4);
- at least one bacterial strain selected from the genera *Propionibacterium, Veillonella, Coprococcus* and *Megasphaera* (A5);
- at least one bacterial strain of the genus *Collinsella* or *Roseburia* (A7);
- at least one bacterial strain selected from the genera *Phascolarctobacterium, Flavonifractor* and *Dialister* (A8); and
- optionally at least one bacterial strain selected from the genera *Alistipes, Bacteroides, Barnesiella, Ruminococcus* and *Prevotella* (A10);
- optionally at least one bacterial strain selected from the genera *Coprococcus, Eubacterium, Flavonifractor and Flintibacter* (A11);
- optionally at least one bacterial strain selected from the genera *Bacteroides, Barnesiella, Bifidobacterium,* (only tryptamine producers), *Enterococcus, Faecalibacterium, Lactobacillus* and *Ruminococcus* (only tryptamine producers) (A12);
- optionally at least one bacterial strain selected from the genera *Anaerostipes,* and *Faecalibacterium* (A13)
- optionally at least one bacterial strain selected from the genera *Bacteroides, Bifidobacterium, Faecalibacterium, Lactobacillus, Prevotella* and *Ruminococcus* (A14); and
- optionally at least one bacterial strain selected from the genera *Akkermansia, Bacteroides, Bifidobacterium* and *Ruminococcus* (A15).

More specifically, the consortium comprises or essentially consists in *Eubacterium limosum* (A6 + A9) and:
- *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans, Eubacterium eligens* and any combination thereof (A1),
- *Faecalibacterium prausnitzii, Anaerostipes hadrus, Roseburia intestinalis Eubacterium ramulus, Eubacterium rectale* and any combination thereof (A2),
- *Lactobacillus rhamnosus, Streptococcus salivarius, Escherichia coli, Lactococcus lactis, Enterococcus faecalis, Enterococcus caccae* and any combination thereof (A3),
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburia hominis* and any combination thereof (A4),
- *Clostridium aminovalericum, Clostridium celatum, Clostridium lactatifermentans, Clostridium neopropionicum, Clostridium propionicum, Megasphaera elsdenii, Veillonella montpellierensis, Coprococcus catus, Veillonella ratti* and any combination thereof (A5);
- *Collinsella aerofaciens, Collinsella intestinalis, Collinsella stercoris, Roseburia hominis* and any combination thereof (A7),
- *Phascolarctobacterium faecium, Dialister succinatiphilus, Flavonifractor plautii, Dialister propionifaciens* and any combination thereof (A8); and
- optionally *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Barnesiella viscericola, Clostridium butyricum, Clostridium bartlettii, Ruminococcus callidus, Ruminococcus flavefaciens, Prevotella copri, Prevotella stercorea, Alistipes finegoldii, Alistipes onderdonkii, Alistipes shahii* and any combination thereof (A10)
- optionally *Clostridium butyricum, Coprococcus eutactus, Eubacterium hallii, Flavonifractor plautii and Flintibacter butyricum* and any combination thereof (A11);
- optionally *Bacteroides caccae, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Clostridium sporogenes, Lactobacillus bulgaricus-52* and *Ruminococcus gnavus* as tryptamine producers, *Acidaminococcus intestini, Bacteroides massiliensis, Bacteroides stercoris, Enterococcus faecalis, Enterococcus faecium* and *Faecalibacterium prausnitzii* as putrescine producers, and *Clostridium bolteae* as spermidine producers and any combination thereof (A12)
- optionally *Anaerostipes caccae, Clostridium bolteae, Clostridium scindens, Clostridium symbiosum* and *Faecalibacterium prausnitzii* and any combination thereof (A13)
- optionally *Bacteroides fragilis, Bifidobacterium adolescentis, Bifidobacterium pseudocatenulatum, Clostridium bolteae, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetaiotaomicron, Bifidobacterium bifidum, Ruminococcus gnavus and Ruminococcus torques* and any combination thereof (A15).

Particularly, the consortium comprises or essentially consists in *Eubacterium limosum* (A6 + A9) and:
- *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans, Eubacterium eligens* and any combination thereof (A1),
- *Faecalibacterium prausnitzii, Anaerostipes hadrus, Roseburia intestinalis Eubacterium ramulus, Eubacterium rectale* and any combination thereof (A2),
- *Lactobacillus rhamnosus, Streptococcus salivarius, Escherichia coli, Lactococcus lactis, Enterococcus faecalis, Enterococcus caccae* and any combination thereof (A3),
- *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum, Roseburia hominis* and any combination thereof (A4),
- *Megasphaera elsdenii, Veillonella montpellierensis, Coprococcus catus, Veillonella ratti* and any combination thereof (A5);
- *Collinsella aerofaciens, Collinsella intestinalis, Collinsella stercoris, Roseburia hominis* and any combination thereof (A7),
- *Phascolarctobacterium faecium, Dialister succinatiphilus, Flavonifractor plautii, Dialister propionifaciens* and any combination thereof (A8); and
- optionally *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Barnesiella viscericola, Ruminococcus callidus, Ruminococcus flavefaciens, Prevotella copri, Prevotella stercorea, Alistipes finegoldii, Alistipes onderdonkii, Alistipes shahii* and any combination thereof (A10)
- optionally *Coprococcus eutactus, Eubacterium hallii, Flavonifractor plautii and Flintibacter butyricum* and any combination thereof (A11);
- optionally *Bacteroides caccae, Bacteroides faecis, Bacteroides fragilis, Bacteroides massiliensis, Bacteroides ovatus, Bacteroides uniformis, Bacteroides vulgatus, Barnesiella intestinihominis, Bifidobacterium adolescentis* and *Lactobacillus plantarum* as GABA producers, *Lactobacillus bulgaricus-*52 and *Ruminococcus gnavus* as tryptamine producers, *Acidaminococcus intestini, Bacteroides massiliensis, Bacteroides stercoris, Enterococcus faecalis, Enterococcus faecium* and *Faecalibacterium prausnitzii* as putrescine producers and any combination thereof (A12)
- optionally *Anaerostipes caccae,* and *Faecalibacterium prausnitzii* and any combination thereof (A13)
- optionally *Bacteroides fragilis, Bifidobacterium adolescentis, Bifidobacterium pseudocatenulatum, Faecalibacterium prausnitzii, Lactobacillus plantarum, Prevotella copri and Ruminococcus lactaris* and any combination thereof (A14); and
- optionally *Akkermansia muciniphila, Bacteroides fragilis, Bacteroides thetaiotaomicron, Bifidobacterium bifidum, Ruminococcus gnavus and Ruminococcus torques* and any combination thereof (A15).

In pure culture, the functions of single bacteria strains (A1) to (A**) may be bidirectional. For example, (A7) may either produce or consume formate. However, when combined in the inventive compositions, the bacteria strains show the properties discussed herein, consuming intermediate metabolites (succinate, lactate, formate) and producing end metabolites (acetate, propionate, butyrate). In one embodiment, the end metabolites are predominantly produced. In a further embodiment the end metabolites are almost exclusively produced, meaning that intermediate metabolites are not found in higher concentrations than 5, 10 or 15 mM each.

Component (i) may be described as a synthetic and symbiotic consortium which is characterized by a combination of microbial activities forming a trophic chain from complex fiber metabolism to the canonical final SCFAs (Short chain fatty acids) found in the healthy intestine: acetate, propionate and butyrate. The trophic completeness of component (i) prevents the accumulation of potentially toxic or pain inducing products such as H₂, lactate, formate and succinate. Activities are screened by functional characterization on different substrates of the human gut microbiota. However, type and origin of strains can be selected according to the targeted level of complexity of the synthetic and symbiotic consortia in order to recompose a complex microbiota replacing FMT. The different bacteria strains (A1) to (A**), particularly (A1) to (A9), grow as a consortium, ensuring degradation of complex polysaccharides usually found in the gut (resistant starch, xylan, arabinoxylan and pectin), reutilization of sugars released, removal of O₂ traces for maintenance of anaerobiosis essential for growth, production of key intermediate metabolites (acetate, lactate, formate, CO2 and H2), reutilization of all intermediate metabolites and production of end metabolites found in a healthy gut (acetate, propionate and butyrate). The microbial symbiotic consortia exclusively produce end-fermentation products that are beneficial and used by the host for different functions such as acetate (energy source for heart and brain cells), propionate (metabolized by the liver) and butyrate (the main source of energy for intestinal epithelial cells).

The combination of strains as discussed herein is chosen to:
1. Degrade the main energy sources in the gut including fibers and intermediate metabolites (all groups, A1- A**);
2. Protect anaerobiosis by reduction of the eventual O₂ through respiration (A3);
3. Produce the main end metabolites found in the intestine (A1, A2, A3, A4, A5, A9);
4. Prevent the enrichment of intermediate metabolites (A5, A6, A7, A8, A9).

The combination of strains from the functional groups (A1) to (A**), in particular (A1) to (A9), encompass the key functions of fiber degradation by the microbiome as described by Lacroix and Chassard in 2013 and results, if cultured together, in a trophic chain analog to the healthy intestinal microbiome in its capacity to exclusively produce end metabolites from complex carbohydrates without accumulation of intermediate metabolites. It is particularly beneficial that the combination of strains from the functional groups (A1) to (A9) prevents the enrichment of intermediate metabolites independent of the composition of the recipient's microbiome and the relative concentration of the enriched intermediate metabolites.

### Component (ii), intermediate metabolites:

The inventive compositions comprise intermediate metabolites in very low concentrations. The low concentration of intermediate metabolites is important to achieve the pharmaceutical beneficial effects discussed below. It is important to note these low concentrations of intermediate metabolites in the context of high concentrations of end metabolites.

Advantageously, the amount of succinate is below 15, 10 or 5 mM, preferably below 2 mM, much preferably below 1 mM.

Advantageously, the amount of formate is below 15, 10 or 5 mM, preferably below 2 mM, much preferably below 1 mM.

Advantageously, the amount of lactate is below 15, 10 or 5 mM, preferably below 2 mM, much preferably below 1 mM.

The concentration may be determined using HPLC-RI. This method has a detection limit of about 1 mM. A reference to below 1 mM thus also refers to a concentration below detection limit.

Preferably, the intermediate metabolites present in the composition are the one produced by the bacteria of the consortium according to the invention. This means that no exogenous source of intermediate metabolites is added in the composition according to the invention. Intermediate metabolites are degraded or convert by the bacteria of the consortium according to the invention. This means that no intermediate depletion other than bacteria conversion is performed during the process of manufacturing.

### Component (iii), end metabolites:

The inventive compositions comprise end metabolites in high concentrations. The high concentration of end metabolites is important to achieve the pharmaceutical beneficial effects discussed below. It is important to note these high concentrations of end metabolites in the context of low concentrations of intermediate metabolites.

Advantageously, the amount of acetate is above 10 mM, preferably above 15, 20, 25 or 30 mM, much preferably above 50 mM. Advantageously, the amount of acetate is below 120 mM.

Advantageously, the amount of propionate is above 2 mM, preferably above 5 mM, much preferably above 10, 15, 20, 25 or 30 mM. Advantageously, the amount of propionate is below 60 mM.

Advantageously, the amount of butyrate is above 2 mM, preferably above 5 mM, much preferably above 10, 15, 20, 25 or 30mM. Advantageously, the amount of butyrate is below 60 mM.

The concentration may be determined using the same methods as discussed above for intermediate metabolites.

Preferably, the end metabolites present in the composition are the one produced by the bacteria of the consortium according to the invention. This means that no exogenous source of end metabolites is added in the composition according to the invention.

### Component (iv), dispersing medium:

The compositions as described herein comprise a dispersing medium (iv). Such medium (iv) is added for a variety of reasons. First, the dispersing medium particularly ensures that bacteria (i) remain as viable live bacteria. Further, the dispersing medium guarantees growth of all groups (A1) - (A**) in the desired ratios. Still further, the dispersing medium plays an important role in recovery of the bacteria strains after storage. A broad range of solid or liquid dispersing media are known and may be used in the context of the present invention.

Suitable media (iv) include liquid media and solid supports. Liquid media generally comprise water and may thus also be termed aqueous media. Such liquid media may comprise a culture medium, a cryoprotective medium and / or a gel forming medium. Solid media may comprise a polymeric support.

Cryoprotecting media are known in the field and include liquid compositions that allow freezing of bacteria strains essentially maintaining their viability. Suitable cryoprotecting agents may be identified by the skilled person, glycerol may be named by way of example. Inventive compositions comprising cryoprotecting agent are typically present as a suspension. Suitable amounts of cryoprotecting media may be determined by the skilled person in routine experiments; suitable are 5-50%, preferably 10-40%, such as 30%. In one embodiment, the cryoprotecting medium comprises glycerol, preferably technical or industrial grade (i.e. comprising at least 95, 96, 97, 98 or 99% glycerol). Preferably, glycerol is present in 10, 20, 30, 40, or 50 %.

Culture media are known in the field and include liquid compositions that allow the growth of bacterial strains. Typically, culture media include a carbon source (glucose, galactose, maltose, lactose, sucrose, fructose, cellobiose), "fibers" (preferably pectine, arabinogalactan, beta-glucan, soluble starch, resistant starch, fructo-oligosacharides, galacto-oligosacharides and, xylan, arabinoxylans, cellulose), proteins (preferably yeast extract, casein, skimmed milk, peptone), co-factors (short chain fatty acids, hemin, FeSO4), vitamins (preferably biotin, cobalamin (B12), 4-aminobenzoic acid, folic acid, pyridoxamine hydrochloride), minerals (preferably sodium bicarbonate, potassium phosphate dibasic, potassium phosphate monobasic, sodium chloride, ammonium sulfate, magnesium sulfate, calcium chloride) and reducing agents (preferably cysteine, titanium(III)-citrate, yeast extract, sodium thioglycolate, dithiothreitol, sodium sulphide, hydrogen sulphite, ascorbate).

In particular, culture media include simple sugars carbon (glucose, galactose, maltose, lactose, sucrose, fructose, cellobiose), "fibers" (preferably pectin, arabinogalactan, beta-glucan, soluble starch, resistant starch, fructo-oligosacharides, galacto-oligosacharides and, xylan, arabinoxylans, cellulose), proteins (preferably yeast extract, casein, skimmed milk, peptone), co-factors (short chain fatty acids, hemin, FeSO4), vitamins (preferably biotin or D-(+)-Biotin (Vit. H), Cobalamin (Vit. B12), 4-aminobenzoic acid or p-aminobenzoic acid (PABA), folic acid (Vit. B11/B9), pyridoxamine hydrochloride (Vit. B6)), minerals (preferably sodium bicarbonate, potassium phosphate dibasic, potassium phosphate monobasic, sodium chloride, ammonium sulfate, magnesium sulfate, calcium chloride) and reducing agents (preferably cysteine, titanium(III)-citrate, yeast extract, sodium thioglycolate, dithiothreitol, sodium sulphide, hydrogen sulphite, ascorbate), guar gum, glycerol, potato starch, rice starch, pea starch, corn starch, wheat starch, inulin, succinate, formate, lactate, iron sulfate, tryptone, fucose, acetate, mucus, trehalose, mannitol, polysorbate and any combination thereof.

Culture media can also comprise compound(s) preventing microbial contamination, e.g. antibiotics selected to be inefficient on desired bacteria.

The skilled person can easily adjust the culture medium to the nutritional requirements of the bacteria to be cultivated.

Inventive compositions comprising culture media are typically present as a suspension.

Gel forming media are known in the field and include compositions comprising a gelling agent. Suitable gelling agents include polymers such as agar, alginate, carrageenan, cellulose and its derivatives, collagen, gelatin, epoxy resin, photo cross-linkable resins, polyacrylamide, polyester, polystyrene and polyurethane, polyacrylamide gel, alginate gel, k-carrageenan, photo-cross-linkable resins, xanthan or gellan.

In one embodiment, the culture medium comprises glycerol. Indeed, the inventors have shown that glycerol has a beneficial effect on butyrate production. Particularly, glycerol in the culture medium may serve as organic carbon source for bacteria, especially butyrate producers such as bacteria of functional group A2 and/or A6.

In an advantageous embodiment, the pharmaceutical compositions are free of, or essentially free of other viable, live bacteria strains.

In a further advantageous embodiment, the pharmaceutical compositions are free of, or essentially free of intermediate metabolites (succinate, formate and lactate).

In a further advantageous embodiment, the pharmaceutical compositions further comprise a culture medium, preferably a culture medium as disclosed herein.

Such pharmaceutical compositions may be formulated according to known principles and adapted to various modes of administration. In one embodiment, the inventive pharmaceutical compositions are adapted to rectal administration. In one further embodiment, the inventive pharmaceutical compositions are adapted to oral administration.

This disclosure also provides for pharmaceutical compositions adapted for personalized medicine, thereby targeting diseases with associated microbiota dysbiosis to specific patient groups or individuals. Bacteria showing similar functionalities but different taxonomic identities can be replaced and exchanged in component (i) used for treatment according to the loss of bacteria detected in patients or specific indications. Loss in diversity and functionality can be targeted for the first time, since the consortium approach allows the controlled re-establishment of single niches in the patient's gut. For example, the engraftment of a formate producing *Bifidobacterium* will be guaranteed by the combination with the formate utilizing *Blautia* strain such as *Blautia* strain in order to avoid enrichment of the intermediate metabolite, that would lead to the elimination of both strains.

This specification further discloses compositions comprising co-cultivated viable, live, human, intestinal bacteria strains A1) to (A**) as defined herein, for use as a pharmaceutical.

### 4. Manufacturing

Manufacturing methods described herein are known and disclosed in WO2018189284.

The manufacturing of compositions as defined herein, comprises the step of co-cultivating bacteria strains (A1) to (A**), particularly (A1) to (A9) optionally with one or more bacteria strains from (A10) to (A15). This manufacturing method ensures the presence of viable bacteria strains (A1) to (A**), particularly (A1) to (A9) in suitable concentrations, and simultaneously the low concentration of intermediate metabolites, as well as the high concentration of end metabolites in the inventive compositions. Further, this method allows manufacturing compositions comprising a defined mix of bacteria strains (A1) to (A**), particularly (A1) to (A9).

The step of co-cultivation, denoted as step (b) herein, is typically complemented by a preceding step (a), providing the bacteria strains (A1) to (A9) / to (A**), optionally with one or more bacteria strains from (A10) to (A15). Further, step (b) may be complemented by post-treatments, as defined in step (c). Thus, the method advantageously comprises the steps of: (a) providing compositions comprising viable live bacteria strains (A1) to (A9) / to (A**) as defined herein, optionally with one or more bacteria strains from (A10) to (A15); (b) co-cultivating the compositions of step (a); and optionally (c) post treatment steps.

**Providing individual bacteria strains, step (a):** Providing a consortium of bacteria strains to a co-cultivation step is known per se and may be applied to the individual strains (A1) to (A9) / to (A**) in a known manner, optionally with one or more bacteria strains from (A10) to (A15).

Advantageously, step (a) comprises two individual steps (a1) and (a2). First, bacteria strains (A1) to (A9) / to (A**), optionally with one or more bacteria strains from (A10) to (A15), are provided and separately cultivated. Second, the materials obtained in (a1) are combined and co-cultivated until intermediate metabolites succinate, formate and lactate are each below 5 mM (a2).

Advantageously, step (a1) is performed batchwise.

Advantageously, cultivation of step (a1) is performed in the presence of a substrate specific for each of said bacteria strains (A1) to (A9) / to (A**), optionally with one or more bacteria strains from (A10) to (A15).

Advantageously, intestinal bacteria strains previously isolated and characterized from one or more healthy donors are used in this step (a1).

Advantageously, step (a2) is performed batchwise.

Advantageously, co-cultivation comprises a first step of batch cultivation followed by a step of continuous cultivation.

Advantageously, cultivation of step (a2) is performed in the presence of peptone, yeast extract, monosaccharides, disaccharides, arabinogalactan, fructo-oligosaccharides, glycerol, fibres, soluble starch, resistant starch, xylan, minerals, co-factors or vitamins and reducing agents; typically at a pH 5.0 - 7.0 and under oxygen-free atmosphere.

Advantageously, step (a2) is terminated, once intermediate metabolites succinate, formate and lactate are each below 5 mM, preferably each below 2mM, much preferred each below 1mM.

**Co-cultivation of bacteria strains, step (b):** As discussed herein, the co-cultivation of step (b) is considered a key element for successful manufacturing the inventive compositions and to obtain the pharmaceutical results described. It is apparent that step (b) follows step (a2).

Such co-cultivation may be performed according to known principles, commercially available equipment (such as a bioreactor) and considering the bacteria strains to be cultivated.

Suitable culture media thus comprise peptone, yeast extract, monosaccharides, disaccharides, arabinogalactan, fructo-oligosaccharides, glycerol, fibers, soluble starch, resistant starch, xylan, minerals, co-factors or vitamins and reducing agents as described above.

Co-cultivation is typically performed at a pH 5.0 - 7.0, preferably at pH 6.5.

Co-cultivation is typically performed under an oxygen free atmosphere, such as CO2 or nitrogen, preferably CO2.

Co-cultivation is typically performed at constant temperature, typically 37°C.

Advantageously, co-cultivation is performed continuously typically at a dilution rate of 0.01 - 1.0 h⁻¹. Accordingly, the co-cultivation of step (b) may be performed in a bioreactor using continuous fermentation and the conditions described herein.

The co-cultivation as described herein provides bacteria consortia in a specific balanced, symbiotic composition, thereby providing for a synergistic effect.

**Post-treatment, step (c):** Post treatment of bacteria consortia, e.g. as obtained from a bioreactor, is known to the skilled person. The material obtained in step (b) is typically present in the form of a suspension, said suspension comprising components (i), (ii) and (iii) as defined herein, dispersed in an aqueous medium that may contain further components, such as non-used substrates, pH modifiers and/or cryoprotectants. This initially obtained product may be directly used in the pharmaceutical applications as described herein, which is considered a significant advantage of the inventive method. Alternatively, the initially obtained product may be subjected to post-treatment steps, such as those described herein. Such post-treatment particularly aims in improving product quality, storage stability, and / or alternative pharmaceutical formulations.

In one embodiment, step (c) comprises stabilization of the product by adding a cryoprotectant. The specification discloses a manufacturing method wherein in step (c) the mixture of strains comprising (A1) to (A9) / to (A**), optionally with one or more bacteria strains from (A10) to (A15), is combined with a cryoprotectant.

In another embodiment, step (c) comprises stabilization of the product after washing the bacteria strains, removing medium residues and metabolic products and further processing of the obtained bacterial biomass by dispersing in an aqueous medium that may contain further components, such as pH modifiers and / or cryoprotectants. Suitable cryoprotectants thus comprise culture media as described above, glycerol, riboflavin, sucrose or dimethyl sulfoxide.

Stabilization of the bacterial biomass can be obtained by freezing with liquid nitrogen, gradual freezing, lyophilization and subsequent storage at temperatures ranging from +4 °C to -80 °C.

In summary, this section describes the production of new consortia based on their trophic interaction and their capacity to combine key functions of the intestinal ecosystem, particularly for reversion of dysbiosis as a consequence of CRC and to treat CRC. For this strategy, the assembly of microbial consortia is not based on the phylogenic distribution of the microbes in healthy intestinal microbiota, but relying on the functionality of key microbial groups. The presented consortia are designed on a function-based rationale in order to restore or re-balance the lacking or dysbalanced functions in dysbalanced intestinal microbiomes, i.e. dysbiosis.

### 5. Other Cancer therapeutics

As outlined above, the inventive compositions may be used in combination with other cancer therapeutics. Such cancer therapeutics are known per se and are described in more detail below. Cancer therapeutics may be grouped by their function into Chemotherapeutic agents, Cancer Immunotherapy Agents, angiogenesis inhibitors and antibiotics. It is understood that this grouping may result in some overlap; e.g. chemotherapeutic agents may be at the same time antibiotics.

**Chemotherapeutic agents** are known and include, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphor-amide, triethiylenethiophosphoramide and trimethylolomel-amine; acetogenins (particularly bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; asarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall; dynemicin, (e.g. dynemicin A); bisphosphonates (e.g. clodronate); an esperamicin; as well as neocarzinostatin chromophore and related chromoprotein enediyne antibiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolinodoxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil; folic acid analogues (e.g. denopterin), methotrexate, pteropterin, trimetrexate; purine analogs (e.g. fludarabine, 6-mercaptopurine, thiamiprine, thioguanine); pyrimidine analogs (e.g. ancitabine, azacitidine, 6-azauridine, carmofur, cytarabine, dideoxyuridine, doxifluridine, enocitabine, floxuridine); androgens (e.g. calusterone, dromostanolone propionate, epitiostanol, mepitiostane, testolactone); anti-adrenals (e.g. aminoglutethimide, mitotane, trilostane); folic acid replenisher (e.g. frolinic acid); aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofuran; spirogermanium; tenuazonic acid; triaziquone; 2,2',2"-trichlorotriethylamine; trichothecenes (e.g. T-2 toxin, verracurin A, roridin A and anguidine); urethan; vindesine; dacarbazine; mannomustine; mitobronitol; mitolactol; pipobroman; gacytosine; arabinoside; cyclophosphamide; thiotepa; taxoids, (e.g. paclitaxel and doxetaxel); chlorambucil; gemcitabine; 6-thioguanine; mercaptopurine; methotrexate; platinum coordination complexes (e.g. cisplatin, oxaliplatin and carboplatin); vinblastine; platinum; etoposide; ifosfamide; mitoxantrone; vincristine; vinorelbine; novantrone; teniposide; edatrexate; daunomycin; aminopterin; xeloda; ibandronate; irinotecan (e.g. CPT-11); topoisomerase inhibitor RFS 2000; difluoromethylomithine (DMFO); retinoids such as retinoic acid; capecitabine; and pharmaceutically acceptable salts, acids or derivatives of any of the above.

**Cancer Immunotherpy Agents** are known and may be selected from the class of checkpoint inhibitors, cancer vaccines, cytokines, cell therapy, CAR-T cells and dentritic cell therapy.

Non-limiting examples of immunotherapies are checkpoint inhibitors include Nivolumab (BMS, anti-PD-1), Pembrolizumab (Merck, anti-PD-1), Ipilimumab (BMS, anti-CTLA-4), MEDI4736 (AstraZeneca, anti-PD-LI), and MPDL3280A (Roche, anti-PD-LI). Other immunotherapies may be tumor vaccines, such as Gardail, Cervarix, BCG, sipulencel-T, Gpl00:209-217, AGS-003, DCVax-L, Algenpantucel-L, Tergenpantucel-L, TG4010, ProstAtak, Prostvac-V/R-TRICOM, Rindopepimul, E75 peptide acetate, IMA901, POL-103A, Belagenpumatucel-L, GSK1572932A, MDX-1279, GV1001, and Tecemotide. Immunotherapy may be administered via injection (e.g., intravenously, intratumorally, subcutaneously, or into lymph nodes), but may also be administered orally, topically, or via aerosol. Immunotherapies may comprise adjuvants such as cytokines.

In some embodiments, the immunotherapy agent is an immune checkpoint inhibitor. Immune checkpoint inhibition broadly refers to inhibiting the checkpoints that cancer cells can produce to prevent or downregulate an immune response. Examples of immune checkpoint proteins include, but are not limited to, CTLA4, PD-1, PD-L1, PD-L2, A2AR, B7-H3, B7-H4, BTLA, KIR, LAG3, TIM-3 or VISTA. Immune checkpoint inhibitors can be antibodies or antigen binding fragments thereof that bind to and inhibit an immune checkpoint protein. Examples of immune checkpoint inhibitors include, but are not limited to, nivolumab, pembrolizumab, pidilizumab, AMP-224, AMP-514, STI-A11 10, TSR-042, RG-7446, BMS-936559, MEDI-4736, MSB-0020718C, AUR-012 and STI-A1010.

In certain embodiments, immune checkpoint inhibitors can be an inhibitory nucleic acid molecule (e.g. an siRNA molecule, an shRNA molecule or an antisense RNA molecule) that inhibits expression of an immune checkpoint protein that inhibits expression of an immune checkpoint protein.

**Angiogenesis inhibitors** are known and include Bevacizumab, Ziv-aflibercept, Sorafenib, Sunitinib, Pazopanib, Regorafenib, and Cabozantinib.

**Antibiotics** are known and include the antibiotics mentioned above. Further, antibiotics may be selected from the group consisting of aminoglycosides, ansamycins, carbacephems, carbapenems, cephalosporins, glycopeptides, lincosamides, lipopeptides, macrolides, monobactams, nitrofurans, oxazolidinones, penicillins, polypeptide antibiotics, quinolones, fluoroquinolone, sulfonamides, tetracyclines, and anti-mycobacterial compounds, as well as combinations thereof.

Novel modalities applied in microbiome therapies such as therapies using phage, or phage like particles, DNA modifying, transferring or transcription silencing techniques and genetically modified bacteria can be used in combination with the composition disclosed herein.

### 6. Carrier and excipients

In some embodiments of the post-treatment steps is for providing a pharmaceutical composition. Such pharmaceutical compositions may be formulated according to known principles and adapted to various modes of administration. In one embodiment, the inventive pharmaceutical compositions are adapted to rectal administration. In one further embodiment, the inventive pharmaceutical compositions are adapted to oral administration.

In one embodiment, the pharmaceutical composition is to be administered orally. For oral administration, the pharmaceutical composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

Well-known thickening agents may also be added to compositions such as corn starch, agar, natural or synthetic gums, resins, methylcellulose, sodium carboxymethylcellulose, guar, xanthan and the like. Preservatives may also be included in the composition, including methylparaben, propylparaben, benzyl alcohol and ethylene diamine tetraacetate salts.

Pharmaceutical compositions as described herein may be formulated to release the active ingredients substantially immediately upon administration or at any predetermined time or time period after administration.

In one embodiment, the pharmaceutical composition further comprises prebiotics. Prebiotics include, but are not limited to, amino acids, biotin, fructo-oligosaccharide, galacto-oligosaccharides, hemicelluloses (e.g., arabinoxylan, xylan, xyloglucan, and glucomannan), inulin, chitin, lactulose, mannan oligosaccharides, oligofructose-enriched inulin, gums (e.g., guar gum, gum arabic and carra geenan), oligofructose, oligodextrose, tagatose, resistant maltodextrins (e.g., resistant starch), trans-galactooligosaccharide, pectins (e.g., xylogalactouronan, citrus pectin, apple pectin, and rhamnogalacturonan-I), dietary fibers (e.g., soy fiber, sugarbeet fiber, pea fiber, corn bran, and oat fiber) and xylooligosaccharides.

In one embodiment, the pharmaceutical composition is to be administrated in a transmucosal way. For transmucosal administration, nasal sprays, rectal or vaginal suppositories can be used. The active compounds can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Preferably, the composition is in a gastro-resistant oral form allowing the bacteria contained in the composition, and more particularly the consortium according to the invention, to pass the stomach and be released into the intestine. Alternatively, the enteric material is acid stable and labile at basic pH, which means that it does not dissolve in the stomach, but dissolves in the intestine. The material that can be used in enteric coatings includes, for example, alginic acid, cellulose acetate phthalate, plastics, waxes, shellac and fatty acids (e.g. stearic acid or palmitic acid).

The composition of the excipient or carrier can be modified as long as it does not significantly interfere with the pharmacological activity of the consortium according to the invention.

Preferably, the pharmaceutical composition comprises an effective therapeutic amount of the consortium according to the invention, preferably 10³ to 10¹⁴ CFU (colony forming units) of bacteria per ml or mg of the pharmaceutical composition.

### Examples

To further illustrate the invention, the following examples are provided. These examples are provided with no intend to limit the scope of the invention.

### Example 1: Rationale, functional groups

Bacterial strains were isolated from healthy donors using Hungate anaerobic culturing techniques (Bryant, 1972) and characterized for growth and metabolite production on M2GSC Medium (ATCC Medium 2857) and modifications thereof whereby the carbon sources glucose, cellobiose and starch were replaced by specific substrates including intermediate metabolites and fibers found in the human intestine. Metabolites produced were quantified using HPLC-RI. The concentrations of the metabolites were quantified by refractive index detection HPLC (Thermo Scientific Accela^{™}, ThermoFisher Scientific) analysis was performed using a SecurityGuard Cartridges Carbo-H (4 x 3.0 mm) (Phenomenex, Torrence, USA) as guard-column connected to a Rezex ROA-Organic Acid H+ column (300 x 7.8 mm) (Phenomenex). Bacterial cultures to be analyzed were centrifuged at 14.000-x g for 10 min at 4 °C. Filter-sterilized (0.45 µL) supernatants were analyzed. Injection volume for each sample was 40 µL. HPLC was run at 40°C with a flow rate of 0.4 mL / min and using H2SO4 (10 mM) as eluent. Peaks were analyzed using AgilentEzChrome Elite software (Version: 3.3.2 SP2, Agilent Technologies, Inc. Pleasanton, USA). Clusters were formed based on substrate usage and metabolite production. Functional groups were defined as combinations of substrate-utilization and metabolite-production as described in claim 1. 9 strains were selected within those clusters in order to assemble the core intestinal carbohydrate metabolism and result in an exclusive production of end metabolites, i.e. acetate, propionate and butyrate, without accumulation of intermediate metabolites, i.e. formate, succinate, lactate.

As outlined above, the combination of bacteria strains is chosen to:
5. Degrade the main energy sources in the gut including fibers and intermediate metabolites (all groups, A1- A**)
6. Protect anaerobiosis by reduction of the eventual O₂ through respiration (A3)
7. Produce the main end metabolites found in the intestine (A1, A2, A3, A4, A5, A9)
8. Prevent the enrichment of intermediate metabolites (A5, A6, A7, A8, A9) independent of the composition of the recipient's microbiome.

For group (A1), *Ruminococcus bromii* was cultivated in YCFA medium (Duncan, Hold, Harmsen, Stewart, & Flint, 2002) for 48 hours using the Hungate technique (Bryant, 1972) resulting in the production of formate (>15 mM) and acetate (>10 mM) as quantified by HPLC-RI.

For group (A2), *Faecalibacterium prausnitzii* was cultivated in M2GSC medium (ATCC Medium 2857) for 48 hours using the Hungate technique (Bryant, 1972) resulting in the consumption of acetate (>10 mM) and in the production of formate (>20 mM) and butyrate (>15 mM) as quantified by HPLC-RI.

For group (A3), *Lactobacillus rhamnosus* was cultivated in MRS Broth (Oxoid) for 48 hours using the Hungate technique resulting in the production of lactate (>50 mM) and formate (>10 mM) as quantified by HPLC-RI.

For group (A4), *Bifidobacterium adolescentis* was cultivated in YCFA medium (Duncan et al., 2002) for 48 hours using the Hungate technique (Bryant, 1972) resulting in the production of acetate (>50 mM), formate (>15 mM) and lactate (> 5 mM) as quantified by HPLC.

For group (A5), *Clostridium lactatifermentans* was cultivated in modified M2-based medium (ATCC Medium 2857) supplemented with DL-lactate [60 mM] instead of a carbohydrate source for 48 hours using the Hungate technique resulting in the consumption of lactate (at least 30 mM) and in the production of propionate (>30 mM), acetate (>10 mM) as detected by HPLC-RI.

For group (A6), *Eubacterium limosum* was cultivated in YCFA medium (Duncan et al., 2002) for 48 hours using the Hungate technique (Bryant, 1972) resulting in the production of acetate (>10 mM) and butyrate (>5 mM) as quantified by HPLC-RI.

For group (A7), *Collinsella aerofaciens* was cultivated in YCFA medium (Duncan et al., 2002) for 48 hours using the Hungate technique resulting in the production of formate (+23.71 mM), lactate (+19.18 mM) and acetate (+16.33 mM) as quantified by HPLC-RI.

For group (A8), *Phascolarctobacterium faecium* was cultivated in M2-based medium (ATCC Medium 2857) supplemented with succinate (60 mM) as sole carbohydrate source for 48 hours using the Hungate technique (Bryant, 1972) resulting in the full consumption of succinate (60 mM) and in the production of propionate (60 mM) as quantified by HPLC-RI.

For group (A9), *Blautia hydrogenotrophica* was cultivated in anaerobic AC21 medium (Leclerc, Bernalier, Donadille, & Lelait, 1997) for >75 hours using the Balch type tubes resulting in the production of acetate (>20 mM) as quantified by HPLC-RI, and consumption of hydrogen.

The combination of strains from the functional groups (A1) - (A9) encompass key functions of the microbiome and results, if cultured together, in a trophic chain analog to the healthy intestinal microbiome in its capacity to exclusively produce end metabolites from complex carbohydrates without accumulation of intermediate metabolites.

### Example 2: assembly of consortium

In order to establish the 9 strains forth on named PB002 in a growing and metabolically interacting manner, a previously validated model for anaerobic intestinal fermentations (Zihler et al., 2013) was adapted using a simplified medium based on YCFA (DSMZ Media N° 1611). Thereby, the 5 g of glucose that are the carbon source in YCFA were replaced by 2 g / L of pectin (Sigma Aldrich), 1 g / L of fructo-oligosacharaides (FB97, Cosucra), 3g/L of potato starch (Sigma Aldrich), and 2g/L of corn starch (Sigma Aldrich). A 200 ml bioreactor (Infors HT) was inoculated with a mix of overnight cultures of all 9 strains and inoculated anaerobically at a 1/100 dilution. The bioreactor was consecutively operated at pH 6.5 for 24 h in order to allow growth of primary degraders and sub sequential consumption of the produced intermediate metabolites. Growth was monitored by base consumption, and optical density of the bioreactor. Metabolites were monitored using HPLC-RI as described above. After the first batch-fermentation, new medium was fed by removing half of total volume and refilling with medium to the original volume of 200 ml in the bioreactor. After two 24-hour batch fermentations the metabolic profile did not contain any intermediate metabolites and more than 40 mM acetate and over 5 mM of propionate and butyrate each. From the end of the second batch fermentation on, the bioreactor was operated continuously at a volume of 200 ml, a flow rate of 12.5 ml / h and a pH of 6.5. Subsequently, a stable metabolic profile established within 7 days after inoculation containing exclusively the desired end metabolites of acetate, propionate and butyrate without detection of intermediate metabolites showing constant production of all desired metabolites without washout of any functional group.

PB002 could therefore be cultured in a bioreactor and showed the desired properties of the intestinal microbiome, i.e. degradation of fibers and proteins into exclusively end-metabolites, a clear indication that the desired interactions and metabolic activities described in example 1 were established in a continuously operated bioreactor.

### Example 3: mouse model/tumor counts

In order to test the potential of PB002 to be used as therapeutic to prevent / treat CRC, PB002 was compared to *Akkermansia muciniphila* and *Peptostreptococcus stomatis* on an AOM-DSS mouse model. This is a well accepted model for colorectal cancer whereby colon carcinogenesis is induced by intraperitoneal (i.p.) injections of azoxymethane (AOM) followed by repeated induction of intestinal inflammation using dextran sodium sulphate (DSS). The AOM/DSS model closely resembles the development of CRC in humans with the typical crypt foci-adenoma- carcinoma sequence. Tumors mainly occur in the distal colon, which is also the primary location for spontaneous CRC in humans (Boivin et al., 2003; De Robertis et al., 2011).

AOM is metabolized into methylazoxymethanol (MAM) in the liver, intestinal epithelium and by intestinal microbes. MAM is a highly carcinogenic compound that forms DNA adducts resulting in miss-match mutations in fast replicating cells such as intestinal epithelial cells. DSS in turn triggers damage of the intestinal barrier, causing IBD-like colitis and after DSS removal hyper-proliferation of IEC occurs as a result of wound healing processes. The combination of DSS-induced intestinal inflammation with mutations triggered via AOM application results in the fast and reproducible formation of colon tumors within 10 and 20 weeks, while amount and size of tumors depends on the mouse strain and AOM/DSS concentration (De Robertis et al., 2011).

In our experiments, C57BI/6J mice were subjected to three cycles of DSS treatment (7 days with 2% of DSS in the drinking water, followed by 10 days of recovery, each). At day 1 and day 9 of each DSS cycle, mice were injected i.p. with 10 mg / kg AOM. All treatment groups were gavaged on 3 consecutive days (day 7 - 9) of each AOM-DSS cycle with 200 µL of the respective suspensions. The following groups of 5 mice each were included:
- the negative control treated with DSS but no AOM (1);
- the positive control gavaged with PBS (2);
- the group treated with *Akkermansia muciniphila* (3);
- the group treated with *Peptostreptococcus stomatis* (4);
- the group treated with PB002 (5).

Groups (3) and (4) contain bacteria repeatedly reported to promote or correlate with cancer, specifically CRC, in the used mouse model and in epidemiological studies of humans (Tsoi et al, 2017; Wong et al 2017; Routy et al, 2017).

Three weeks after the last AOM-DSS cycle, tumor load was determined by mouse endoscopy (Fig. 1). Mice treated with PB002 (5) showed significantly less tumor counts compared to treatment groups (3) and (4). The data therefore, indicates that PB002 might prevent tumor formation in the colon.

### Example 4: mouse model / analysis of immune response

To study the effect of PB002 application on immune responses in the intestine flow cytometry (LSR Fortessa Cytometer, Becton Dickinson) was performed on intestinal lamina propria cells from the mice described in example 3. The following markers were used to discriminate immune cell subsets: CD45, CD3, CD4, CD8, TCRgd, FoxP3, TCRb, N1.1, F4/80, MHC-II, CD11c, PD1, PD-L1, and PD-L2.

These markers allowed discriminating between different T cell subsets, NK cells, macrophages and dendritic cells. Inclusion of PD1, PD-L1, and PD-L2 further allowed investigating a possible involvement of immune checkpoint molecules - molecules that suppress anti-cancer immune responses.

These analyses clearly revealed that treatment with PB002 resulted in a reduction of F4/80+ macrophages when compared to *Akkermansia-* (3), *Peptostreptococcus-* (4) or PBS-treated (2) control mice, but the abundance of NK1.1 expressing NK cells was increased (Fig. 2). Of note, certain macrophages, especially those expressing the checkpoint inhibitor molecules PD-L1 and PD-L2 can reduce anti-tumor immune response, while NK cells can promote tumor eradication and directly kill tumor cells.

To further elucidate an involvement of checkpoint inhibitors in the observed phenotype, we analyzed expression of the checkpoint molecule PD-L1 on non-T cells, as well as PD1 on CD8+ T cells. In *Akkermansia-* (3) or *Peptostreptococcus-treated* (4) mice, abundance of PD-L1 expressing cells was not altered or even enhanced when compared to the control. In PB002-treated mice (5) in contrast, frequency of PD-L1 expressing cells was reduced (Fig. 3A), indicating that in these mice, suppression of anti-tumor immune responses is abolished. In *Akkermansia-* (3) and *Peptostreptococcus-treated* (4) mice, frequencies of PD1+ cytotoxic T cells were increased (Figure 3B), indicating a suppression of anti-tumor T cell activity. Of note, PB002 treatment (5) did not promote PD1 expression in CD8+ T cells.

### References

Throughout this specification, the following references are cited:
1. Boivin, G. P., Washington, K., Yang, K., Ward, J. M., Pretlow, T. P., Russell, R., Besselsen, D. G., Godfrey, V. L., Doetschman, T., Dove, W. F., Pitot, H. C., Halberg, R. B., Itzkowitz, S. H., Groden, J., Coffey, R. J (2003). Pathology of mouse models of intestinal cancer: consensus report and recommendations. Gastroenterology, 124, 762-777.
2. Bryant, M. P. (1972). Commentary on the Hungate technique for culture of anaerobic bacteria. The American Journal of Clinical Nutrition, 25(12), 1324-1328.
3. Chassard, C. & Lacroix, C. (2013). Carbohydrates and the human gut microbiota. Current opinion in clinical nutrition and metabolic care, 16(4), 453-460.
4. De Robertis, M., Massi, E., Poeta, M. L., Carotti, S., Morini, S., Cecchetelli, L., Signori, E., Fazio, V. M. (2011). The AOM/DSS murine model for the study of colon carcinogenesis: From pathways to diagnosis and therapy studies. Journal of Carcinogenesis, 10, 9.
5. Duncan, S. H., Hold, G. L., Harmsen, H. J. M., Stewart, C. S., & Flint, H. J. (2002). Growth requirements and fermentation products of Fusobacterium prausnitzii, and a proposal to reclassify it as Faecalibacterium prausnitzii gen. nov., comb. nov. International Journal of Systematic and Evolutionary Microbiology, 52(6), 2141-2146.
6. Leclerc, M., Bernalier, A., Donadille, G., & Lelait, M. (1997). H2/CO2 metabolism in acetogenic bacteria isolated from the human colon. Anaerobe, 3(5), 307-315.
7. Ritari, J., Salojärvi, J., Lahti, L., & de Vos, W. M. (2015). Improved taxonomic assignment of human intestinal 16S rRNA gene sequences by a dedicated reference database. BMC Genomics, 16(1056).
8. Routy, B., Le Chatelier, E., Derosa, L., Duong, C. P. M., Alou, M. T., Daillère, R., ... Zitvogel, L. (2018). Gut microbiome influences efficacy of PD-1-based immunotherapy against epithelial tumors. Science (New York, N.Y.), 359(6371), 91-97.
9. Tsoi, H., Lam, K. C., Dong, Y., Zhang, X., Lee, C. K., Zhang, J., ... Yu, J. (2017). Pre-45s rRNA promotes colon cancer and is associated with poor survival of CRC patients. Oncogene, 36(44), 6109-6118.
10. Wong, S. H., Zhao, L., Zhang, X., Nakatsu, G., Han, J., Xu, W., ... Yu, J. (2017). Gavage of Fecal Samples From Patients With Colorectal Cancer Promotes Intestinal Carcinogenesis in Germ-Free and Conventional Mice. Gastroenterology, 153(6), 1621-1633.
11. Zihler, A., Fuentes, S., Dostal, A., Payne, A. N., Vazquez Gutierrez, P., Chassard, C., Grattepanche F., de Vos W. M., Lacroix, C. (2013). Novel Polyfermentor Intestinal Model (PolyFermS) for Controlled Ecological Studies: Validation and Effect of pH. PloS One, 8(10).

## Claims

1. A composition comprising viable, live bacteria strains (i), intermediate metabolites (ii), end metabolites (iii), and a dispersing medium (iv), for use as a medicament to treat colorectal cancer (CRC) and / or intestinal microbiome dysbiosis related to CRC treatment, **characterized in that** said bacteria strains (i) are selected from:
- (A1) strains consuming sugars, fibers, and resistant starch, producing formate and acetate, and being selected from the genera *Ruminococcus, Clostridium, Dorea* and *Eubacterium,* optionally selected from the genera *Ruminococcus, Dorea* and *Eubacterium*;
- (A2) strains consuming sugars, starch and acetate, producing formate and butyrate, and being selected from the genera *Faecalibacterium, Roseburia, Eubacterium* and *Anaerostipes,* optionally selected from the genera *Faecalibacterium, Roseburia* and *Anaerostipes*;
- (A3) strains consuming sugars and oxygen, producing lactate, and being selected from the genera *Lactobacillus, Streptococcus, Escherichia, Lactococcus* and *Enterococcus*;
- (A4) strains consuming sugars, starch, and carbon dioxide, producing lactate, formate and acetate, and being selected from the genera *Bifidobacterium* and *Roseburia,* optionally being of the genus *Bifidobacterium*;
- (A5) strains consuming lactate or proteins, producing propionate and acetate, and being selected from the genera *Clostridium, Propionibacterium, Veillonella, Coprococcus* and *Megasphaera,* optionally selected from the genera *Clostridium, Propionibacterium, Veillonella* and *Megasphaera*;
- (A6) strains consuming lactate and starch, producing acetate, butyrate and hydrogen, and being selected from the genera *Eubacterium, Clostridium* and *Anaerostipes*;
- (A7) strains consuming sugar, starch, and formate, producing lactate, formate and acetate, and being selected from the genera *Collinsella* and *Roseburia,* optionally being of the genus *Collinsella*;
- (A8) strains consuming succinate, producing propionate and acetate, and being selected from the genera *Phascolarctobacterium, Flavonifractor* and *Dialister,* optionally selected from the genera *Phascolarctobacterium* and *Dialister*;
- (A9) strains consuming sugars, fibers, formate and hydrogen, producing acetate and optionally butyrate and being selected from the genera *Acetobacterium, Blautia, Clostridium, Moorella, Eubacterium, Methanobrevibacter, Methanomassiliicoccus* and *Sporomusa,* optionally selected from the genera *Acetobacterium, Blautia, Clostridium, Moorella, Methanobrevibacter, Methanomassiliicoccus* and *Sporomusa*; and
- optionally (A10) strains consuming sugars, fibers, and resistant starch, producing succinate, and being selected from the genera *Alistipes, Bacteroides, Blautia, Barnesiella, Clostridium, Ruminococcus* and *Prevotella,* optionally selected from the genera *Alistipes, Bacteroides, Blautia, Clostridium, Ruminococcus* and *Prevotella,* preferably *Alistipes, Bacteroides, Barnesiella, Ruminococcus* and *Prevotella*;
wherein said bacteria strains are in each case identified through classification of the full 16S gene with assignment for the different taxonomic levels Phylum: 75%, Class: 78.5%, Order: 82%, Family: 86.5%, Genus: 94.5%, sequence similarity;
wherein bacteria strains (i) of all nine groups (A1) to (A9) are present and bacteria strains of group (A10) are optionally present;
wherein said bacteria strains (i) are present in a total concentration of over 10⁹ bacteria per ml composition; and have a viability of over 50%, preferably over 70%, as determined by flow cytometry;
wherein said intermediate metabolites (ii) are selected from: succinate in an amount of less than 5 mM, formate in an amount of less than 5 mM, and lactate in an amount of less than 5 mM; and
wherein said end metabolites (iii) are selected from: acetate in an amount of at least 10 mM, propionate in an amount of at least 2 mM, and butyrate in an amount of at least 2 mM; and
wherein said dispersing medium (iv) is preferably selected from: culture media, cryoprotecting media, aqueous gels, and polymeric supports.

2. The composition for use of claim 1 for use as a medicament to treat CRC.

3. The composition for use of claim 1 for use as a medicament to treat intestinal microbiome dysbiosis related to CRC treatment.

4. The composition for use according to any one of claims 1 to 3, wherein said treatment includes the prevention, the treatment, and / or the delay of progression of said disease.

5. The composition for use according to any one of claims 1 to 4 for use as a mono therapy.

6. The composition for use according to any one of claims 1 to 4 for use in combination therapy.

7. The composition for use according to claim 6 in combination with another cancer therapeutic, preferably a cancer therapeutic selected from the group of chemotherapeutic agents; cancer immunotherapy agents (particularly checkpoint inhibitors, cancer vaccines, cytokines, cell therapy, CAR-T cells and dendritic cell therapy); angiogenesis inhibitors; and antibiotics.

8. The composition for use according to any one of the preceding claims, **characterized in that** each of said viable, live bacteria strains (i) is present in an amount of 10⁵-10¹⁴ 16S rRNA gene copies per ml, as quantified by qPCR.

9. The composition for use according to any one of the preceding claims, **characterized in that** said composition further comprises one or several bacterial strains selected from (A11) strains consuming proteins and producing acetate or butyrate, and being selected from the genera *Clostridium, Coprococcus, Eubacterium, Flavonifractor and Flintibacter*;
(A12) strains consuming proteins, fibers, starches or sugars and producing biogenic amines such as y-aminobutyric acid (GABA), cadaverine, dopamine, histamine, putrescine, serotonin, spermidine and/or tryptamine, and being selected from the genera *Bacteroides, Barnesiella, Bifidobacterium, Clostridium* (only tryptamine producers), *Enterococcus, Faecalibacterium, Lactobacillus* and *Ruminococcus* (only tryptamine producers);
(A13) strains consuming primary bile acids and producing secondary metabolites, and being selected from the genera *Anaerostipes, Blautia, Clostridium* and *Faecalibacterium*;
(A14) strains producing vitamins such as cobalamin (B12), folate (B9) or riboflavin (B2), and being selected from the genera *Bacteroides, Bifidobacterium, Blautia, Clostridium, Faecalibacterium, Lactobacillus, Prevotella* and *Ruminococcus*;
(A15) strains consuming mucus and being selected from the genera *Akkermansia, Bacteroides, Bifidobacterium* and *Ruminococcus.*

10. The composition for use according to any one of the preceding claims, **characterized in that** said viable bacteria strains (i) are such that
- (A1) are selected from *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans and Eubacterium eligens,* optionally selected from *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans and Eubacterium eligens*;
- (A2) are selected from *Faecalibacterium prausnitzii, Anaerostipes hadrus, Roseburia intestinalis Eubacterium ramulus* and *Eubacterium rectale,* optionally selected from *Faecalibacterium prausnitzii, Anaerostipes hadrus,* and *Roseburia intestinalis*;
- (A3) are selected from *Lactobacillus rhamnosus, Streptococcus salivarius, Escherichia coli, Lactococcus lactis, Enterococcus faecalis* and *Enterococcus caccae,* optionally selected from *Lactobacillus rhamnosus, Streptococcus salivarius, Escherichia coli, Lactococcus lactis* and *Enterococcus caccae*;
- (A4) are selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum,* and *Roseburia hominis,* optionally selected from *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum* and *Bifidobacterium pseudocatenulatum;*
- (A5) are selected from *Clostridium aminovalericum, Clostridium celatum, Clostridium lactatifermentans, Clostridium neopropionicum, Clostridium propionicum, Megasphaera elsdenii, Veillonella montpellierensis, Coprococcus catus* and *Veillonella ratti,* optionally selected from *Clostridium aminovalericum, Clostridium celatum, Clostridium lactatifermentans, Clostridium neopropionicum, Clostridium propionicum, Megasphaera elsdenii, Veillonella montpellierensis* and *Veillonella ratti;*
- (A6) are selected from *Anaerostipes caccae, Clostridium indolis, Eubacterium hallii, Eubacterium limosum,* and *Eubacterium ramulus;*
- (A7) are selected from *Collinsella aerofaciens, Collinsella intestinalis, Collinsella stercoris, and Roseburia hominis,* optionally selected from *Collinsella aerofaciens, Collinsella intestinalis* and *Collinsella stercoris;*
- (A8) are selected from *Phascolarctobacterium faecium, Dialister succinatiphilus, Flavonifractor plautii* and *Dialister propionifaciens,* optionally selected from *Phascolarctobacterium faecium, Dialister succinatiphilus* and *Dialister propionifaciens*;
- (A9) are selected from *Acetobacterium carbinolicum, Acetobacterium malicum,* Acetobacterium wieringae, *Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii* and *Candidatus Methanomassiliicoccus intestinalis,* optionally selected from *Acetobacterium carbinolicum, Acetobacterium malicum,* Acetobacterium wieringae, *Blautia hydrogenotrophica, Blautia producta, Clostridium aceticum, Clostridium glycolicum, Clostridium magnum, Clostridium mayombe, Methanobrevibacter smithii* and *Candidatus Methanomassiliicoccus intestinalis; and*/*or*
- (A10) are selected from *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Barnesiella viscericola, BlautialClostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii,* optionally from *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* and *Alistipes shahii,* preferably from *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis* , *Barnesiella viscericola, Ruminococcus callidus, Ruminococcus flavefaciens, Prevotella copri, Prevotella stercorea, Alistipes finegoldii, Alistipes onderdonkii,* and *Alistipes shahii.*

11. The composition for use according to any one of the preceding claims, **characterized in that** said viable bacteria strains (i) are either
*Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6), *Collinsella aerofaciens* (A7), *Phascolarctobacterium faecium* (A8), and *Blautia hydrogenotrophica* (A9) and optionally *Bacteroides xylanisolvens* (A10); or
*Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6 and A9), *Collinsella aerofaciens* (A7) and *Phascolarctobacterium faecium* (A8) and optionally *Bacteroides xylanisolvens* (A10).

12. The composition for use according to any one of the preceding claims, wherein component (iv) is selected from: cryoprotecting media comprising glycerol; and/or culture media comprising peptone, yeast extract, monosaccharides, disaccharides, arabinogalactan, fructo-oligosaccharides, fibres, glycerol, soluble starch, resistant starch, xylan, minerals, co-factors, vitamins and reducing agents.

13. The composition for use according to any one of the preceding claims, which is free of, or essentially free of, other viable, live bacteria.

14. The composition for use according to any one of the preceding claims, which is free of, or essentially free of, succinate, formate and lactate.

15. The pharmaceutical composition for use according to any one of the preceding claims, which is adapted to rectal administration or oral administration.

## Patentansprüche

1. Zusammensetzung, die lebensfähige, lebende Bakterienstämme (i), Zwischenmetaboliten (ii), Endmetaboliten (iii) und ein Dispersionsmedium (iv) umfasst, zur Verwendung als Medikament zur Behandlung von kolorektalem Krebs (CRC) und/oder intestinaler Mikrobiom-Dysbiose im Zusammenhang mit der CRC-Behandlung, **dadurch gekennzeichnet, dass** die Bakterienstämme (i) ausgewählt sind aus:
- (A1) Stämme, die Zucker, Fasern und resistente Stärke verbrauchen, Formiat und Acetat produzieren und aus den Gattungen Ruminococcus, Clostridium, Dorea und Eubacterium ausgewählt sind, bevorzugt aus den Gattungen Ruminococcus, Dorea und Eubacterium ausgewählt sind;
- (A2) Stämme, die Zucker, Stärke und Acetat verbrauchen, Formiat und Butyrat produzieren und aus den Gattungen Faecalibacterium, Roseburia, Eubacterium und Anaerostipes ausgewählt sind, bevorzugt aus den Gattungen Faecalibacterium, Roseburia und Anaerostipes ausgewählt sind;
- (A3) Stämme, die Zucker und Sauerstoff verbrauchen, Laktat produzieren und aus den Gattungen Lactobacillus, Streptococcus, Escherichia, Lactococcus und Enterococcus ausgewählt sind;
- (A4) Stämme, die Zucker, Stärke und Kohlendioxid verbrauchen, Laktat, Formiat und Acetat produzieren und aus den Gattungen Bifidobacterium und Roseburia ausgewählt sind, bevorzugt zur Gattung Bifidobacterium gehören;
- (A5) Stämme, die Laktat oder Proteine verbrauchen, Propionat und Acetat produzieren und aus den Gattungen Clostridium, Propionibacterium, Veillonella, Coprococcus und Megasphaera ausgewählt sind, bevorzugt aus den Gattungen Clostridium, Propionibacterium, Veillonella und Megasphaera ausgewählt sind;
- (A6) Stämme, die Laktat und Stärke verbrauchen, Acetat, Butyrat und Wasserstoff produzieren und aus den Gattungen Eubacterium, Clostridium und Anaerostipes ausgewählt sind;
- (A7) Stämme, die Zucker, Stärke und Formiat verbrauchen, Laktat, Formiat und Acetat produzieren und aus den Gattungen Collinsella und Roseburia ausgewählt sind, wobei sie bevorzugt zur Gattung Collinsella gehören;
- (A8) Stämme, die Succinat verbrauchen, Propionat und Acetat produzieren und aus den Gattungen Phascolarctobacterium, Flavonifractor und Dialister ausgewählt sind, bevorzugt ausgewählt sind aus den Gattungen Phascolarctobacterium und Dialister;
- (A9) Stämme, die Zucker, Fasern, Formiat und Wasserstoff verbrauchen, Acetat und gegebenenfalls Butyrat produzieren und aus den Gattungen Acetobacterium, Blautia, Clostridium, Moorella, Eubacterium, Methanobrevibacter, Methanomassiliicoccus und Sporomusa, bevorzugt ausgewählt aus den Gattungen Acetobacterium, Blautia, Clostridium, Moorella, Methanobrevibacter, Methanomassiliicoccus und Sporomusa;
und
- gegebenenfalls (A10) Stämme, die Zucker, Fasern und resistente Stärke verzehren, Succinat produzieren und aus den Gattungen Alistipes, Bacteroides, Blautia, Bamesiella, Clostridium, Ruminococcus und Prevotella ausgewählt sind, bevorzugt ausgewählt sind aus den Gattungen Alistipes, Bacteroides, Blautia, Clostridium, Ruminococcus und Prevotella, vorzugsweise aus Alistipes, Bacteroides, Bamesiella, Ruminococcus und Prevotella;
wobei die Bakterienstämme in jedem Fall durch Klassifizierung des vollständigen 16S-Gens mit Zuordnung zu den verschiedenen taxonomischen Ebenen Phylum: 75%, Class: 78,5%, Ordnung: 82%, Familie: 86,5%, Gattung: 94,5%, Sequenzähnlichkeit identifiziert sind; wobei Bakterienstämme (i) aus allen neun Gruppen (A1) bis (A9) vorhanden sind und Bakterienstämme der Gruppe (A10) optional vorhanden sind;
wobei die Bakterienstämme (i) in einer Gesamtkonzentration von mehr als 10⁹ Bakterien pro ml Zusammensetzung vorhanden sind und eine Lebensfähigkeit von mehr als 50 %, vorzugsweise mehr als 70 %, aufweisen, bestimmbar mittels Durchflusszytometrie;
wobei die Zwischenmetaboliten (ii) ausgewählt sind aus: Succinat in einer Menge von weniger als 5 mM, Formiat in einer Menge von weniger als 5 mM, und Lactat in einer Menge von weniger als 5 mM; und
wobei die Endmetaboliten (iii) ausgewählt sind aus: Acetat in einer Menge von mindestens 10 mM, Propionat in einer Menge von mindestens 2 mM und Butyrat in einer Menge von mindestens 2 mM; und
wobei das Dispersionsmedium (iv) vorzugsweise ausgewählt ist aus: Kulturmedien, Kryoprotektionsmedien, wässrigen Gelen und polymeren Trägern.

2. Die Zusammensetzung zur Verwendung nach Anspruch 1 zur Verwendung als Medikament zur Behandlung von CRC.

3. Die Zusammensetzung zur Verwendung nach Anspruch 1 zur Verwendung als Medikament zur Behandlung der Dysbiose des Darmmikrobioms im Zusammenhang mit der Behandlung von CRC.

4. Zusammensetzung zur Verwendung nach einem der Ansprüche 1 bis 3, wobei die Behandlung die Vorbeugung, die Behandlung und/oder die Verzögerung des Fortschreitens der Krankheit umfasst.

5. Die Zusammensetzung zur Verwendung nach einem der Ansprüche 1 bis 4 zur Verwendung als Monotherapie.

6. Die Zusammensetzung zur Verwendung nach einem der Ansprüche 1 bis 4 zur Verwendung in der Kombinationstherapie.

7. Zusammensetzung zur Verwendung nach Anspruch 6 in Kombination mit einem anderen Krebstherapeutikum, vorzugsweise einem Krebstherapeutikum, ausgewählt aus der Gruppe der Chemotherapeutika, Krebsimmuntherapeutika (insbesondere Checkpoint-Inhibitoren, Krebsimpfstoffe, Zytokine, Zelltherapie, CAR-T-Zellen und dendritische Zelltherapie), Angiogenese-Inhibitoren und Antibiotika.

8. Zusammensetzung zur Verwendung nach einem der vorhergehenden Ansprüche, **dadurch gekennzeichnet, dass** jeder der lebensfähigen, lebenden Bakterienstämme (i) in einer Menge von 10⁵-10¹⁰ 16S rRNA-Genkopien pro ml vorhanden ist, quantifizierbar durch qPCR.

9. Zusammensetzung zur Verwendung gemäß einem der vorhergehenden Ansprüche, **dadurch gekennzeichnet, dass** die Zusammensetzung außerdem einen oder mehrere Bakterienstämme umfasst, die ausgewählt sind aus
(A11) Stämme, die Proteine verbrauchen und Acetat oder Butyrat produzieren und aus den Gattungen Clostridium, Coprococcus, Eubacterium, Flavonifractor und Flintibacter ausgewählt sind;
(A12) Stämme, die Proteine, Fasern, Stärke oder Zucker verzehren und biogene Amine wie gamma-Aminobuttersäure (GABA), Kadaverin, Dopamin, Histamin, Putrescin, Serotonin, Spermidin und/oder Tryptamin produzieren, und ausgewählt sind aus den Gattungen Bacteroides, Bamesiella, Bifidobacterium, Clostridium (nur Tryptaminproduzenten), Enterococcus, Faecalibacterium, Lactobacillus und Ruminococcus (nur Tryptaminproduzenten);
(A13) Stämme, die primäre Gallensäuren verbrauchen und Sekundärmetaboliten produzieren und aus den Gattungen Anaerostipes, Blautia, Clostridium und Faecalibacterium ausgewählt sind;
(A14) Stämme, die Vitamine wie Cobalamin (B12), Folat (B9) oder Riboflavin (B2) produzieren und aus den Gattungen Bacteroides, Bifidobacterium, Blautia, Clostridium, Faecalibacterium, Lactobacillus, Prevotella und Ruminococcus ausgewählt sind;
(A15) Stämme, die Schleim verzehren und aus den Gattungen Akkermansia, Bacteroides, Bifidobacterium und Ruminococcus ausgewählt sind.

10. Zusammensetzung zur Verwendung nach einem der vorhergehenden Ansprüche, **dadurch gekennzeichnet, dass** die lebensfähigen Bakterienstämme (i) derart sind, dass
- (A1) ausgewählt sind aus Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans und Eubacterium eligens, bevorzugt ausgewählt sind aus Ruminococcus bromii, Ruminococcus lactaris, Ruminococcus champanellensis, Ruminococcus callidus, Ruminococcus gnavus, Ruminococcus obeum, Dorea longicatena, Dorea formicigenerans und Eubacterium eligens;
- (A2) ausgewählt sind aus Faecalibacterium prausnitzii, Anaerostipes hadrus, Roseburia intestinalis, Eubacterium ramulus und Eubacterium rectale, bevorzugt ausgewählt sind aus Faecalibacterium prausnitzii, Anaerostipes hadrus und Roseburia intestinalis;
- (A3) ausgewählt sind aus Lactobacillus rhamnosus, Streptococcus salivarius, Escherichia coli, Lactococcus lactis, Enterococcus faecalis und Enterococcus caccae, bevorzugt ausgewählt sind aus Lactobacillus rhamnosus, Streptococcus salivarius, Escherichia coli, Lactococcus lactis und Enterococcus caccae;
- (A4) ausgewählt sind aus Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum und Roseburia hominis bevorzugt ausgewählt sind aus Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum und Bifidobacterium pseudocatenulatum;
- (A5) ausgewählt sind aus Clostridium aminovalericum, Clostridium celatum, Clostridium lactatifermentans, Clostridium neopropionicum, Clostridium propionicum, Megasphaera elsdenii, Veillonella montpellierensis, Coprococcus catus und Veillonella ratti, bevorzugt ausgewählt sind aus Clostridium aminovalericum, Clostridium celatum, Clostridium lactatifermentans, Clostridium neopropionicum, Clostridium propionicum, Megasphaera elsdenii, Veillonella montpellierensis und Veillonella ratti;
- (A6) ausgewählt sind aus Anaerostipes caccae, Clostridium indolis, Eubacterium hallii, Eubacterium limosum und Eubacterium ramulus;
- (A7) ausgewählt sind aus Collinsella aerofaciens, Collinsella intestinalis, Collinsella stercoris und Roseburia hominis, bevorzugt ausgewählt sind aus Collinsella aerofaciens, Collinsella intestinalis und Collinsella stercoris;
- (A8) ausgewählt sind aus Phascolarctobacterium faecium, Dialister succinatiphilus, Flavonifractor plautii und Dialister propionifaciens, bevorzugt ausgewählt sind aus Phascolarctobacterium faecium, Dialister succinatiphilus und Dialister propionifaciens;
- (A9) aus Acetobacterium carbinolicum, Acetobacterium malicum, Acetobacterium wieringae, Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii und Candidatus Methanomassiliicoccus intestinalis ausgewählt sind, bevorzugt ausgewählt sind aus Acetobacterium carbinolicum, Acetobacterium malicum, Acetobacterium wieringae, Blautia hydrogenotrophica, Blautia producta, Clostridium aceticum, Clostridium glycolicum, Clostridium magnum, Clostridium mayombe, Methanobrevibacter smithii und Candidatus Methanomassiliicoccus intestinalis; und/oder
- (A10) ausgewählt sind aus Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Bamesiella viscericola, Blautia/Costridium coccoides, Blautia luti, Blautia wexlerae, Clostridium butyricum, Clostridium bartlettii, Ruminococcus callidus, Ruminococcusflavefaciens, Prevotella copri, Prevotella stercorea, Alistipes finegoldii, Alistipes onderdonkii und Alistipes shahii, bevorzugt ausgewählt sind aus Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Blautia/Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii, und Alistipes shahii, vorzugsweise aus Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Bamesiella intestinihominis , Barnesiella viscericola, Ruminococcus calidus, Ruminococcus flavefaciens, Prevotella copri, Prevotella stercorea, Alistipes finegoldii, Alistipes onderdonkii, und Alistipes shahii.

11. Zusammensetzung zur Verwendung nach einem der vorhergehenden Ansprüche, **dadurch gekennzeichnet, dass** die lebensfähigen Bakterienstämme (i) entweder
Ruminococcus bromii (A1), Faecalibacterium prausnitzii (A2), Lactobacillus rhamnosus (A3), Bifidobacterium adolescentis (A4), Anaerotignum Iactatifermentans (A5), Eubacterium limosum (A6), Collinsella aerofaciens (A7), Phascolarctobacterium faecium (A8), und Blautia hydrogenotrophica (A9) und optional Bacteroides xylanisolvens (A10) sind; oder
Ruminococcus bromii (A1), Faecalibacterium prausnitzii (A2), Lactobacillus rhamnosus (A3), Bifidobacterium adolescentis (A4), Anaerotignum Iactatifermentans (A5), Eubacterium Iimosum (A6 und A9), Collinsella aerofaciens (A7) und Phascolarctobacterium faecium (A8) und optional Bacteroides xylanisolvens (A10) sind.

12. Zusammensetzung zur Verwendung nach einem der vorhergehenden Ansprüche, wobei Komponente (iv) ausgewählt ist aus: Kälteschutzmedien, die Glycerin umfassen; und/oder Kulturmedien, die Pepton, Hefeextrakt, Monosaccharide, Disaccharide, Arabinogalactan, Fructo-Oligosaccharide, Fasern, Glycerin, lösliche Stärke, resistente Stärke, Xylan, Mineralien, Co-Faktoren, Vitamine und Reduktionsmittel umfassen.

13. Die Zusammensetzung zur Verwendung nach einem der vorhergehenden Ansprüche, die frei oder im Wesentlichen frei von anderen lebensfähigen, lebenden Bakterien ist.

14. Zusammensetzung zur Verwendung nach einem der vorhergehenden Ansprüche, die frei oder im Wesentlichen frei von Succinat, Formiat und Lactat ist.

15. Pharmazeutische Zusammensetzung zur Verwendung nach einem der vorhergehenden Ansprüche, die zur rektalen oder oralen Verabreichung geeignet ist.

## Revendications

1. Composition comprenant des souches bactériennes vivantes viables (i), des métabolites intermédiaires (ii), des métabolites finaux (iii), et un milieu de dispersion (iv), pour son utilisation en tant que médicament pour traiter un cancer colorectal (CRC) et/ou une dysbiose du microbiome intestinale associée à un traitement du CRC, **caractérisée en ce que** lesdites souches bactériennes (i) sont choisies parmi :
- les souches (A1) consommant des sucres, des fibres, et de l'amidon résistant, produisant du formate et de l'acétate, et étant choisies parmi les genres *Ruminococcus, Clostridium, Dorea* et Eubacterium, éventuellement choisies parmi les genres *Ruminococcus, Dorea* et *Eubacterium* ;
- les souches (A2) consommant des sucres, de l'amidon et de l'acétate, produisant du formate et du butyrate, et étant choisies parmi les genres *Faecalibacterium, Roseburia, Eubacterium* et *Anaerostipes,* éventuellement choisies parmi les genres *Faecalibacterium, Roseburia et Anaerostipes* ;
- les souches (A3) consommant des sucres et de l'oxygène, produisant du lactate, et étant choisies parmi les genres *Lactobacillus, Streptococcus, Escherichia, Lactococcus* et *Enterococcus* ;
- les souches (A4) consommant des sucres, de l'amidon, et du dioxyde de carbone, produisant du lactate, du formate et de l'acétate, et étant choisies parmi les genres *Bifidobacterium* et *Roseburia,* éventuellement étant du genre *Bifidobacterium* ;
- les souches (A5) consommant du lactate ou des protéines, produisant du propionate et de l'acétate, et étant choisies parmi les genres *Clostridium, Propionibacterium, Veillonella, Coprococcus* et *Megasphaera,* éventuellement choisies parmi les genres *Clostridium, Propionibacterium, Veillonella et Megasphaera ;*
- les souches (A6) consommant du lactate et de l'amidon, produisant de l'acétate, du butyrate et de l'hydrogène, et étant choisies parmi les genres *Eubacterium, Clostridium* et *Anaerostipes ;*
- les souches (A7) consommant du sucre, de l'amidon, et du formate, produisant du lactate, du formate et de l'acétate, et choisies parmi les genres *Collinsella* et *Roseburia,* éventuellement étant du genre *Collinsella* ;
- les souches (A8) consommant du succinate, produisant du propionate et de l'acétate, et étant choisies parmi les genres *Phascolarctobacterium, Flavonifractor* et *Dialister,* éventuellement choisies parmi les genres *Phascolarctobacterium* et *Dialister* ;
- les souches (A9) consommant des sucres, des fibres, du formate et de l'hydrogène, produisant de l'acétate et éventuellement du butyrate, et étant choisies parmi les genres *Acetobacterium, Blautia, Clostridium, Moorella, Eubacterium, Methanobrevibacter, Methanomassiliicoccus* et *Sporomusa,* éventuellement choisies parmi les genres *Acetobacterium, Blautia, Clostridium, Moorella, Methanobrevibacter, Methanomassiliicoccus* et *Sporomusa* ; et
- éventuellement les souches (A10) consommant des sucres, des fibres, et de l'amidon résistant, produisant du succinate, et étant choisies parmi les genres *Alistipes, Bacteroides, Blautia, Barnesiella, Clostridium, Ruminococcus* et *Prevotella,* éventuellement choisies parmi les genres *Alistipes, Bacteroides, Blautia, Clostridium, Ruminococcus* et *Prevotella,* de préférence *Alistipes, Bacteroides, Barnesiella, Ruminococcus* et *Prevotella ;*
dans laquelle lesdites souches bactériennes sont dans chaque cas identifiées par classification du gène 16S complet avec attribution pour les différents niveaux taxonomiques phylum : 75 %, classe : 78,5 %, ordre : 82 %, famille, 86,5 %, genre : 94,5 %, similarité de séquence ;
dans laquelle les souches bactériennes (i) de tous les neuf groupes (A1) à (A9) sont présentes et des souches bactériennes du groupe (A10) sont éventuellement présentes ;
dans laquelle lesdites souches bactériennes (i) sont présentes à une concentration totale de plus de 10⁹ bactéries par ml de composition ; et ont une viabilité supérieure à 50 %, de préférence supérieure à 70 %, telle que déterminée par cytométrie de flux ;
dans laquelle lesdits métabolites intermédiaires (ii) sont choisis parmi : le succinate en une quantité inférieure à 5 mM, le formate en une quantité inférieure à 5 mM, et le lactate en une quantité inférieure à 5 mM ; et
dans laquelle lesdits métabolites finaux (iii) sont choisis parmi : l'acétate en une quantité d'au moins 10 mM, le propionate en une quantité d'au moins 2 mM, et le butyrate en une quantité d'au moins 2 mM ; et
dans laquelle ledit milieu de dispersion (iv) est de préférence choisi parmi : un milieu de culture, un milieu cryoprotecteur, des gels aqueux, et des supports polymères.

2. Composition pour une utilisation selon la revendication 1, destinée à être utilisée en tant que médicament pour traiter un CRC.

3. Composition pour une utilisation selon la revendication 1, destinée à être utilisée en tant que médicament pour traiter une dysbiose du microbiome intestinal associée à un traitement du CRC.

4. Composition pour une utilisation selon l'une quelconque des revendications 1 à 3, dans laquelle ledit traitement comprend la prévention, le traitement, et/ou le retard de la progression de ladite maladie.

5. Composition pour une utilisation selon l'une quelconque des revendications 1 à 4, pour une utilisation en monothérapie.

6. Composition pour une utilisation selon l'une quelconque des revendications 1 à 4, pour une utilisation en thérapie combinée.

7. Composition pour une utilisation selon la revendication 6, en combinaison avec un autre agent thérapeutique anticancéreux, de préférence un agent thérapeutique anticancéreux choisi dans le groupe comprenant les agents chimiothérapeutiques ; les agents immunothérapeutiques anticancéreux (en particulier les inhibiteurs de point de contrôle, les vaccins anticancéreux, les cytokines, une thérapie cellulaire, les cellules CAR-T et une thérapie par cellules dendritiques) ; les inhibiteurs d'angiogenèse ; et les antibiotiques.

8. Composition pour une utilisation selon l'une quelconque des revendications précédentes, **caractérisée en ce que** chacune desdites souches bactériennes vivantes viables (i) est présente en une quantité de 10⁵ à 10¹⁴ copies géniques d'ARNr 16S par ml, telle que quantifiée par qPCR.

9. Composition pour une utilisation selon l'une quelconque des revendications précédentes, **caractérisée en ce que** ladite composition comprend en outre une ou plusieurs souches bactériennes choisies parmi
les souches (A11) consommant des protéines et produisant de l'acétate ou du butyrate, et étant choisies parmi les genres *Clostridium, Coprococcus, Eubacterium, Flavonifractor* et *Flintibacter ;*
les souches (A12) consommant des protéines, des fibres, des amidons ou des sucres et produisant des amines biogéniques telles que l'acide γ-aminobutyrique (GABA), la cadavérine, la dopamine, l'histamine, la putrescine, le sérotonine, la spermidine et/ou la tryptamine, et étant choisies parmi les genres *Bacteroides, Barnesiella, Bifidobacterium, Clostridium* (uniquement les producteurs de tryptamine), *Enterococcus, Faecalibacterium, Lactobacillus* et *Ruminococcus* (uniquement les producteurs de tryptamine) ;
les souches (A13) consommant des acides biliaires primaires et produisant des métabolites secondaires, et étant choisies parmi les genres *Anaerostipes, Blautia, Clostridium* et *Faecalibacterium ;*
les souches (A14) produisant des vitamines telles que la cobalamine (B12), le folate (B9) ou la riboflavine (B2), et étant choisies parmi les genres *Bacteroides, Bifidobacterium, Blautia, Clostridium, Faecalibacterium, Lactobacillus, Prevotella* et *Ruminococcus ;*
les souches (A15) consommant du mucus et étant choisies parmi les genres *Akkermansia, Bacteroides, Bifidobacterium* et *Ruminococcus.*

10. Composition pour une utilisation selon l'une quelconque des revendications précédentes, **caractérisée en ce que** lesdites souches bactériennes viables (i) sont telles que :
- (A1) sont choisies parmi *Ruminococcus bromii, Ruminococcus lactaris, Ruminococcus champanellensis, Ruminococcus callidus, Ruminococcus gnavus, Ruminococcus obeum, Clostridium scindens, Dorea longicatena, Dorea formicigenerans* et *Eubacterium eligens,* éventuellement choisies parmi *Ruminococcus bromii, Ruminococcus lactaris, Ruminococcus champanellensis, Ruminococcus callidus, Ruminococcus gnavus, Ruminococcus obeum, Dorea longicatena, Dorea formicigenerans* et *Eubacterium eligens ;*
- (A2) sont choisies parmi *Faecalibacterium prausnitzii, Anaerostipes hadrus, Roseburia intestinalis, Eubacterium ramulus* et *Eubacterium rectale,* éventuellement choisies parmi *Faecalibacterium prausnitzii, Anaerostipes hadrus,* et *Roseburia intestinalis ;*
- (A3) sont choisies parmi *Lactobacillus rhamnosus, Streptococcus salivarius, Escherichia coli, Lactococcus lactis, Enterococcus faecalis* et *Enterococcus caccae,* éventuellement choisies parmi *Lactobacillus rhamnosus, Streptococcus salivarius, Escherichia coli, Lactococcus lactis* et *Enterococcus caccae ;*
- (A4) sont choisies parmi *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum, Bifidobacterium pseudocatenulatum,* et *Roseburia hominis,* éventuellement choisies parmi *Bifidobacterium adolescentis, Bifidobacterium angulatum, Bifidobacterium bifidum, Bifidobacterium breve, Bifidobacterium catenulatum, Bifidobacterium dentium, Bifidobacterium gallicum, Bifidobacterium longum* et *Bifidobacterium pseudocatenulatum ;*
- (A5) sont choisies parmi *Clostridium aminovalericum, Clostridium celatum, Clostridium lactatifermentans, Clostridium neopropionicum, Clostridium propionicum, Megasphaera elsdenii, Veillonella montpellierensis, Coprococcus catus* et *Veillonella ratti,* éventuellement choisies parmi *Clostridium aminovalericum, Clostridium celatum, Clostridium lactatifermentans, Clostridium neopropionicum, Clostridium propionicum, Megasphaera elsdenii, Veillonella montpellierensis* et *Veillonella* ratti ;
- (A6) sont choisies parmi *Anaerostipes caccae, Clostridium indolis, Eubacterium hallii, Eubacterium limosum,* et *Eubacterium ramulus ;*
- (A7) sont choisies parmi *Collinsella aerofaciens, Collinsella intestinalis, Collinsella stercoris,* et *Roseburia hominis,* éventuellement choisies parmi *Collinsella aerofaciens, Collinsella intestinalis* et *Collinsella stercoris ;*
- (A8) sont choisies parmi *Phascolarctobacterium faecium, Dialister succinatiphilus, Flavonifractor plautii* et *Dialister propionifaciens,* éventuellement choisies parmi *Phascolarctobacterium faecium, Dialister succinatiphilus* et *Dialister propionifaciens* ;
- (A9) sont choisies parmi *Acetobacterium carbinolicum, Acetobacterium malicum, Acetobacterium wieringae, Blautia hydrogenotrophica, Blautia producta, Eubacterium limosum, Eubacterium hallii, Eubacterium ramulus, Clostridium aceticum, Clostridium glycolicum, Clostridium magnum, Clostridium mayombe, Methanobrevibacter smithii* et *Candidatus Methanomassiliicoccus intestinalis,* éventuellement choisies parmi *Acetobacterium carbinolicum, Acetobacterium malicum, Acetobacterium wieringae, Blautia hydrogenotrophica, Blautia producta, Clostridium aceticum, Clostridium glycolicum, Clostridium magnum, Clostridium mayombe, Methanobrevibacter smithii* et *Candidatus Methanomassiliicoccus intestinalis* ; et/ou
- (A10) sont choisies parmi *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Barnesiella viscericola, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* et *Alistipes shahii,* éventuellement parmi *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Blautia*/*Clostridium coccoides, Blautia luti, Blautia wexlerae, Clostridium butyricum, Clostridium bartlettii, Ruminococcus callidus, Ruminococcus flavefaciens, Prevotella copri, Prevotella stercorea, Alistipes finegoldii, Alistipes onderdonkii,* et *Alistipes shahii,* de préférence parmi *Bacteroides faecis, Bacteroides fragilis, Bacteroides ovatus, Bacteroides plebeius, Bacteroides uniformis, Bacteroides thetaiotaomicron, Bacteroides vulgatus, Bacteroides xylanisolvens, Barnesiella intestinihominis, Barnesiella viscericola, Ruminococcus callidus, Ruminococcus flavefaciens, Prevotella copri, Prevotella stercorea, Alistipes finegoldii, Alistipes onderdonkii, etAlistipes shahii.*

11. Composition pour une utilisation selon l'une quelconque des revendications précédentes, **caractérisée en ce que** lesdites souches bactériennes viables (i) sont soit
*Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6), *Collinsella aerofaciens* (A7), *Phascolarctobacterium faecium* (A8), et *Blautia hydrogenotrophica* (A9) et éventuellement *Bacteroides xylanisolvens* (A10) ; soit
*Ruminococcus bromii* (A1), *Faecalibacterium prausnitzii* (A2), *Lactobacillus rhamnosus* (A3), *Bifidobacterium adolescentis* (A4), *Anaerotignum lactatifermentans* (A5), *Eubacterium limosum* (A6 et A9), *Collinsella aerofaciens* (A7) et *Phascolarctobacteriumfaecium* (A8) et éventuellement *Bacteroides xylanisolvens* (A10).

12. Composition pour une utilisation selon l'une quelconque des revendications précédentes, dans laquelle le composant (iv) est choisi parmi : les milieux cryoprotecteurs comprenant du glycérol ; et/ou les milieux de culture comprenant de la peptone, de l'extrait de levure, des monosaccharides, des disaccharides, de l'arabinogalactane, des fructo-oligosaccharides, des fibres, du glycérol, de l'amidon soluble, de l'amidon résistant, du xylane, des minéraux, des cofacteurs, des vitamines et des agents réducteurs.

13. Composition pour une utilisation selon l'une quelconque des revendications précédentes, qui est exempte ou pratiquement exempte d'autres bactéries vivantes viables.

14. Composition pour une utilisation selon l'une quelconque des revendications précédentes, qui est exempte ou pratiquement exempte de succinate, formate et lactate.

15. Composition pour une utilisation selon l'une quelconque des revendications précédentes, qui est adaptée pour une administration par voie rectale ou une administration par voie orale.
